# EUROPEAN PATENT SPECIFICATION

(11) **EP 4 359 023 B1**
(45) Date of publication and mention of the grant of the patent: **05.08.2026**
(21) Application number: 22842642.5
(22) Date of filing: 27.06.2022
(51) Int. Cl.: A61L 27/34, A01N 25/04, A01N 25/10, C07K 7/08, C07K 7/50, A61K 38/03, A61K 38/12, A61P 31/00, A61L 17/00, A61L 17/14, A61L 27/54

(54) **SUBSTRATES MODIFIED WITH PEPTOID-LOADED MICROGELS FOR RESISTANCE TO BACTERIAL COLONIZATION**
MIT PEPTOIDBELADENEN MIKROGELEN MODIFIZIERTE SUBSTRATE ZUR RESISTENZ GEGEN BAKTERIELLE KOLONISATION
SUBSTRATS MODIFIÉS AVEC DES MICROGELS CHARGÉS DE PEPTOÏDES POUR LA RÉSISTANCE À LA COLONISATION BACTÉRIENNE

(30) Priority: 25.06.2021 US 202163214782 P
(43) Date of publication of application: 01.05.2024
(73) Proprietor: Maxwell Biosciences, Inc., Austin, TX 78738 (US)
(72) Inventor: ZHAO, Wenhan, Hoboken, New Jersey 07030 (US); BARRON, Annelise, Redwood City, California 94062 (US); LIBERA, Matthew, Providence, New Jersey 07974 (US)
(74) Representative: Pisani, Diana Jean
(86) International application number: PCT/US2022/035189
(87) International publication number: WO 2023/287570

(56) References cited:
- WO-A1-2020/163462
- WO-A1-2021/127294
- WO-A2-2010/098843
- WO-A2-2023/287570
- US-A1- 2014 011 015
- US-A1- 2014 031 523
- US-A1- 2018 030 209
- US-A1- 2019 040 172
- US-A1- 2019 053 790
- US-A1- 2019 216 513
- US-A1- 2020 203 881
- US-A1- 2020 277 450
- US-A1- 2020 352 990
- WANG QICHEN ET AL: "Self-Assembled Poly(ethylene glycol)- co -Acrylic Acid Microgels to Inhibit Bacterial Colonization of Synthetic Surfaces", APPLIED MATERIALS & INTERFACES, vol. 4, no. 5, 2 May 2012 (2012-05-02), US, pages 2498 - 2506, XP093264761, ISSN: 1944-8244, DOI: 10.1021/am300197m
- WU YONG ET AL: "PEG-Based Microgels to Modify Biomaterials Surfaces", MACROMOLECULAR SYMPOSIA, vol. 329, no. 1, 1 July 2013 (2013-07-01), DE, pages 35 - 40, XP093264749, ISSN: 1022-1360, DOI: 10.1002/masy.201200106
- CHONGSIRIWATANA NATHANIEL P., MILLER TYLER M., WETZLER MODI, VAKULENKO SERGEI, KARLSSON AMY J., PALECEK SEAN P., MOBASHERY SHAHRIA: "Short Alkylated Peptoid Mimics of Antimicrobial Lipopeptides", ANTIMICROBIAL AGENTS AND CHEMOTHERAPY, vol. 55, no. 1, 1 January 2011 (2011-01-01), US , pages 417 - 420, XP093070614, ISSN: 0066-4804, DOI: 10.1128/AAC.01080-10

## Description

### GOVERNMENT RIGHTS

This invention was made with government support under grant # W911NF2010277 awarded by the Army Research Office; grant # DMR-1608406 awarded by the National Science Foundation; grant # 1DP1 OD029517-01 awarded by the U.S. Public Health Services; and grant No. DE-AC02-05CH11231 awarded by the U.S. Department of Energy.

### FIELD OF THE DISCLOSURE

The present disclosure relates generally to surface treatments, and more particularly to methods for imparting resistance to bacterial colonization to substrates through treatment with peptoid-loaded microgels.

### BACKGROUND OF THE DISCLOSURE

Even though surgical operating rooms (ORs) are commonly referred to as being sterile or aseptic, in reality, OR atmospheres contain microbes from many different sources. Such sources include ventilation systems, shedding from clothing, sneezing or coughing by OR personnel, and pedestrian traffic [1]. These airborne bacteria can sediment directly onto the surfaces of an implantable biomedical device once the device is removed from its sterile packaging. Early measurements [2] reported a sedimentation rate of about 3x10⁶ CFU/m²-h. Improved sanitation practices have steadily reduced sedimentation rates to about 10³ to 10⁴ CFU/m²-h [3]. However, these sedimentation rates suggest that, even under these improved conditions, devices are often being implanted after they have been contaminated by hundreds or thousands of bacteria. Once implanted and exposed to favorable growth conditions within the body, a subset of these bacteria may develop into biofilms and can lead to chronic, device-associated infections.

Various implantable devices, such as hip/knee prostheses [4], heart valves [5], pacemakers [6], cochlear implants [7], shunts [8], surgical mesh [9], sutures [10], and tissue-engineering constructs [11], are susceptible to device-associated infection. It is well established that the incidence of surgical site infection increases linearly with time in the OR [12], and there is a strong consensus that intra-operative contamination is responsible for at least some device-associated infections [13].

WO 2010/098843 A2 describes peptoid oligomers demonstrate antimicrobial activity and may be prepared as pharmaceutical compositions and used for the prevention or treatment of a variety of conditions in mammals including humans where microbial invasion is involved. Wang, Q., Uzno 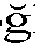 lu, E., Wu, Y., & Libera, M., "Self-Assembled Poly(ethylene glycol)-co-Acrylic Acid Microgels to Inhibit Bacterial Colonization of Synthetic Surfaces", Applied Materials & Interfaces, 2 May 2012, vol, 4, no. 5, pages 2498-2506 explores the use of self-assembled microgels to inhibit the bacterial colonization of synthetic surfaces both by modulating surface cell adhesiveness at length scales comparable to bacterial dimensions (~1 µm) and by locally storing/releasing an antimicrobial. Wu, Y., Wang, Q., & Libera, M., "PEG-based Microgels to Modify Biomaterials Surfaces", Macromolecular Symposia, 1 July 2013, vol. 329, no. 1, pages 35-40, reviews progress in the field of PEG-based microgels, giving examples illustrating their electrostatic deposition onto biomaterial surfaces and their ability to sequester antimicrobials for applications involving biomaterials-associated infection. WO 2021/127294 A1 describes a contraceptive device which includes a (preferably elastomeric) surface; and a lubricant applied to the surface. The lubricant includes (a) a lubricious medium, and (b) a pharmaceutically effective amount of a peptoid disposed in said lubricious medium. The antimicrobial peptoid may impart protection against some common sexually transmitted diseases, while also imparting spermicidal activity to the lubricious medium and contraceptive device.

### SUMMARY OF THE DISCLOSURE

According to the invention there is provided a biomedical device according to claim 1. Certain optional features of such a device are set forth in the dependent claims. The biomedical device is a hip prosthesis, a knee prosthesis, a heart valve, a pacemaker, a cochlear implant, a shunt, a surgical mesh, a suture or a tissue-engineering construct, and comprises a surface selected from the group consisting of metal surfaces, ceramic surfaces and polymeric surfaces. A polyanionic microgel is disposed on said surface, and a peptoid is disposed in said polyanionic microgel.

Also described herein, but not claimed, is a method provided for treating a surface of a biomedical device. The method comprises depositing a polyanionic microgel onto the surface of the biomedical device, and loading the deposited polyanionic microgel with a peptoid.

Also described herein, but not claimed, is a self-defensive surface which comprises a substrate; a polyanionic microgel disposed on said substrate; and a peptoid disposed in said polyanionic microgel.

Also described herein, but not claimed, is a biomedical implant that comprises a substrate; and a release surface disposed on said substrate, wherein said release surface releases a peptoid in response to the presence of a pathogen; wherein said release surface includes a 3D cross-linked colloidal structure.

Also described herein, but not claimed, is a method for treating a surface of a biomedical implant to render it resistant to bacterial cultivation. The method comprises (a) priming a surface of the implant with poly(allylamine hydrochloride), thereby obtaining a primed surface; (b) applying a sub-monolayer of poly(acrylic acid) (PAA) microgels to the primed surface, thereby obtaining a 3D cross-linked colloidal structure; and (c) loading the colloidal structure with a peptoid by forming a complex between the PAA microgels and the peptoid.

### BRIEF DESCRIPTION OF THE DRAWINGS

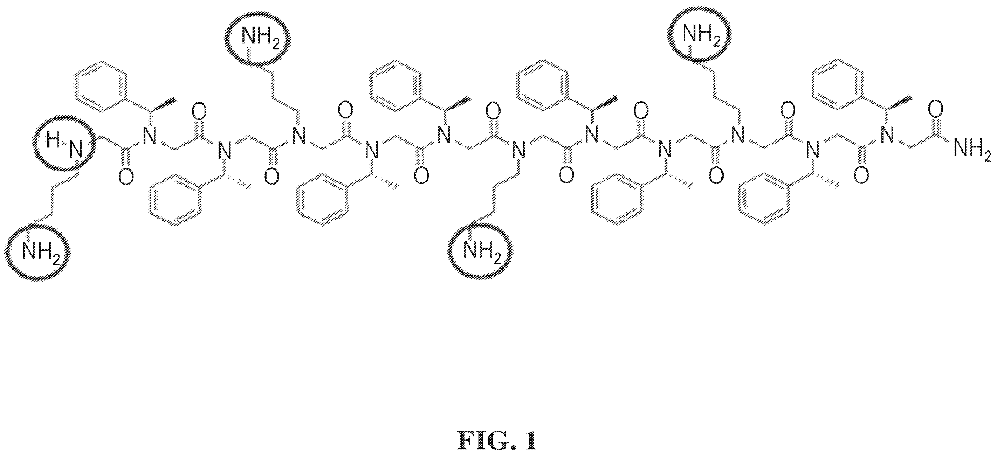

FIG. 1 is an illustration showing the molecular structure of TM1, a 12-mer antimicrobial peptoid with 5 positive charges at physiological pH (circles).

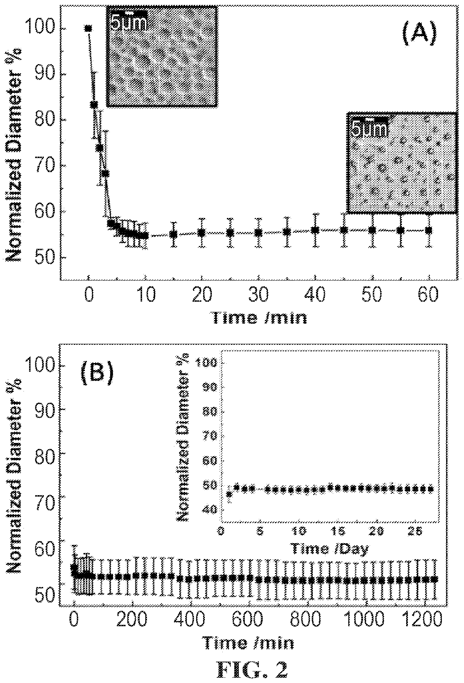

FIG. 2A is a graph showing the time-resolved change in microgel diameter, which indicates TM1 loading (1 mg/mL TM1 in 0.01 M phosphate buffer) by complexation within PAA microgels. The inset optical micrographs show the same set of hydrated microgels before/after loading.
FIG. 2B is a graph illustrating the lack of diameter change in microgels, indicating that the complexed TM1 remains sequestered within the PAA microgels when exposed to TM1-free PBS. Sequestration is maintained for 4 weeks (inset) with daily changes of PBS. Each data point/error bar represents the average/standard deviation from n=5 measurements.

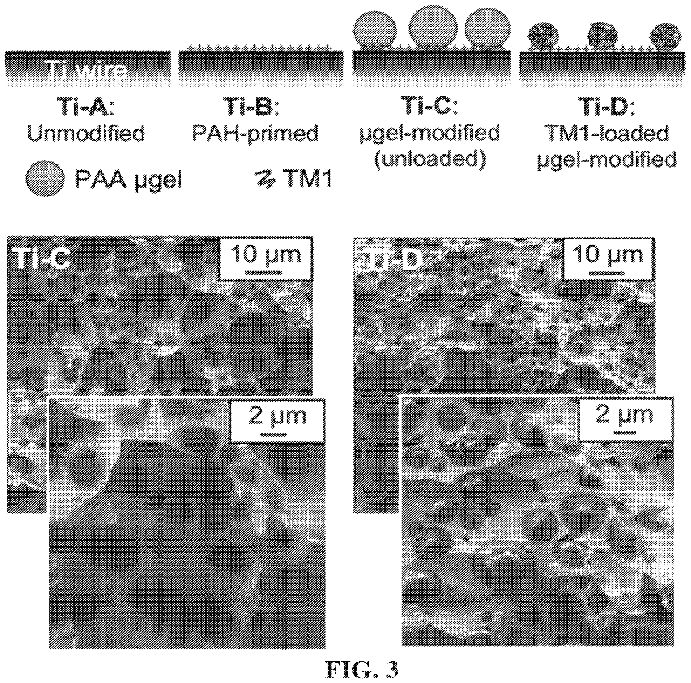

FIG. 3 illustrates the contamination resistance of Ti rods subjected to each of four different surface treatments. The top portion of the figure is a series of schematic illustrations of four surface treatments applied to Ti rods. The bottom portion of the figure is a series of SEM images of the surfaces of the rods modified with unloaded PAA microgels (bottom left) and TM1-loaded PAA microgels (bottom right).

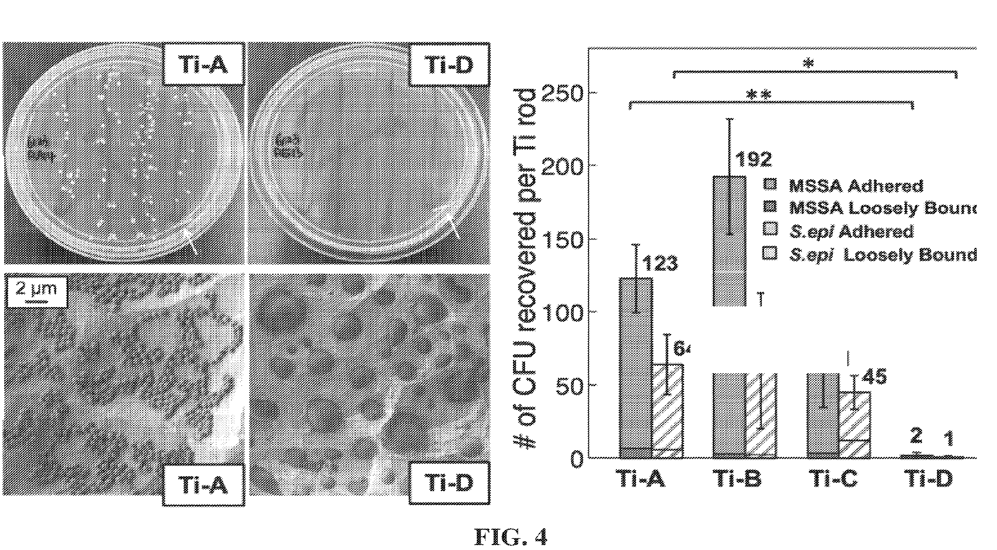

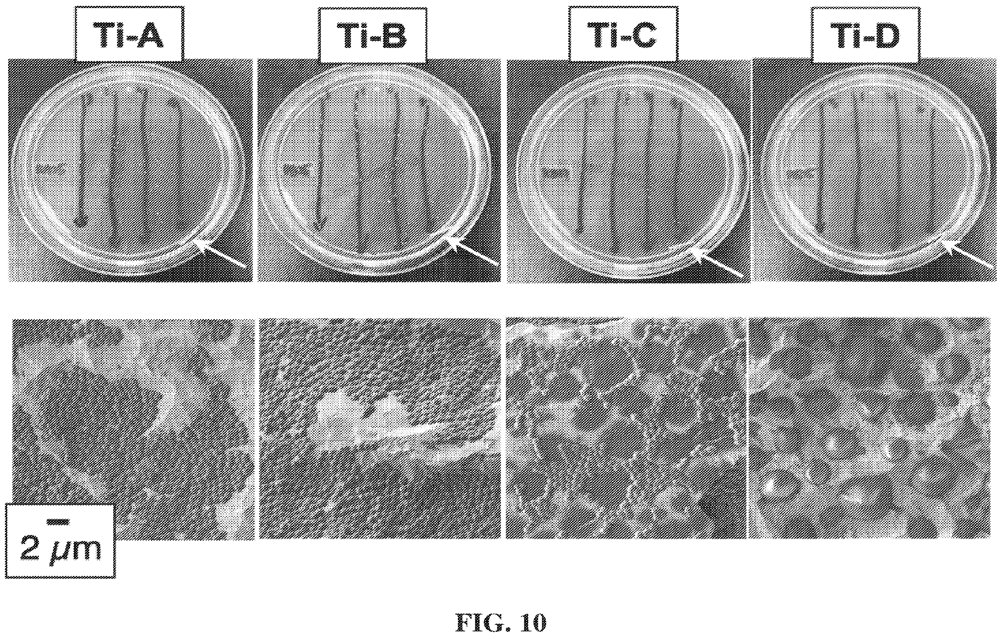

FIG. 4 depicts the results of an assay of colonization resistance. The top left image shows the TSA plate assaying the culturable colonies on a Ti-A (unmodified) rod. The white arrow indicates the rod after rolling four times across the agar surface. The SEM image of the Ti-A rod (bottom left) after culture shows substantial MSSA colonization. The corresponding images for the Ti-D rod (TM1-loaded microgel modified) indicate no culturable colonies. The Ti-D SEM image shows that the loaded microgels remain intact on the rod surface, but MSSA are absent. Images for both MSSA and S. epidermidis contamination for all four (A-D) surface modifications are presented as FIGs. 10-11. The right panel quantifies the total number of loosely bound and strongly adhered MSSA and S. epidermidis CGUs for each of the four conditions. The averages are indicated, and the error bars represent the standard deviation for n=3 samples. * indicates p < 0.05, ** indicates p < 0.005.

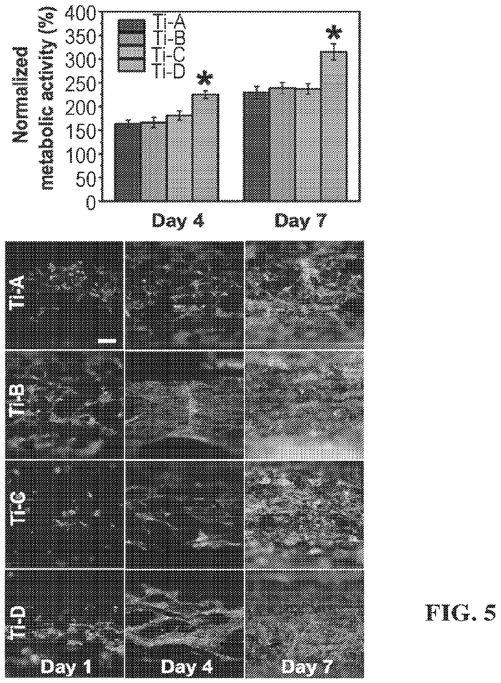

FIG. 5 depicts the results of cytocompatibility assays using human fetal osteoblasts. Metabolic activity of hFOB cells where the average for each surface treatment is normalized to its Day 1 value. The asterisk indicates a statistically significant difference (t-test with p<0.05) between the fully modified Ti-D and the Ti-A unmodified control. (Bottom) Confocal fluorescence images (DAPI/Phalloidin staining) of hFOB cells grown on Ti rods with each of the four surface treatments (the scale bar is 100 µm).

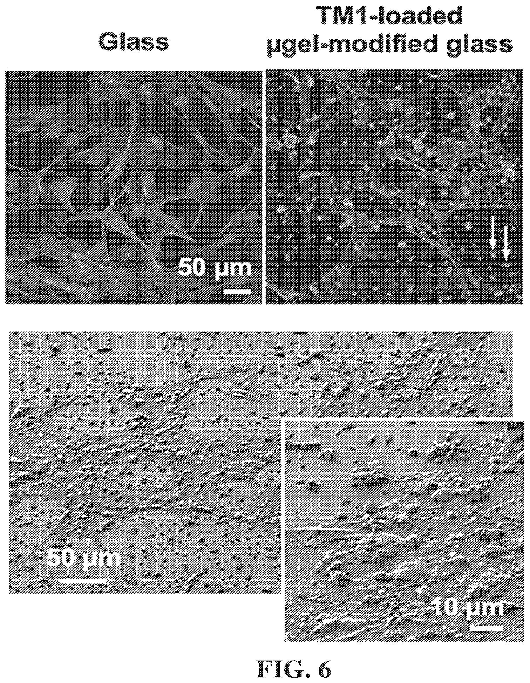

FIG. 6 illustrates how many of the imaging complications associated with the curved and rough surface of Ti rods may be avoided through the use of glass substrates. The series of confocal images shows hFOB cells cultured on unmodified glass (top left) and TM1-loaded microgel-modified glass (top right) after 7 days of culture. The white arrows (top right) indicate auto-fluorescence from TM1-loaded microgels. The lower SEM images indicate that hFOB cells are able to spread over the TM1-loaded microgels.

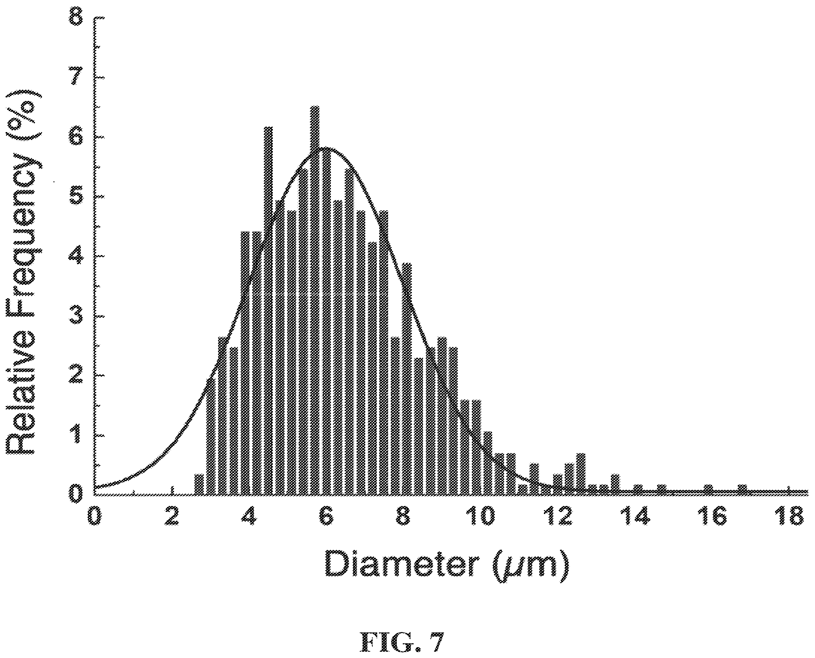

Fig. 7 is a size distribution histogram of PAA microgel diameters when hydrated in 0.01M phosphate buffer (pH 7.4; [Na+] = 0.016 M). The solid line represents a Gaussian distribution fit to the data where the average and standard deviation are 6.0 µm and 2.0 µm, respectively.

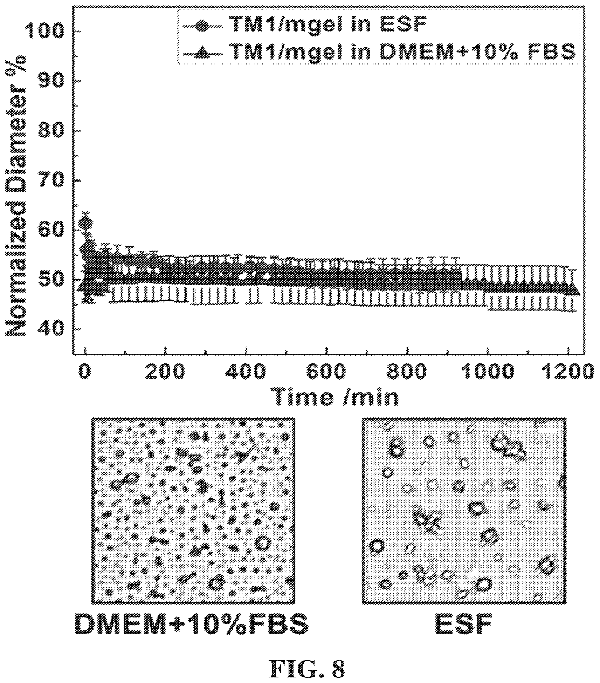

Fig. 8 is a graph and a series of images showing no change in microgel diameter, with TM1 remaining sequestered within PAA microgels, when exposed to serum-containing DMEM or to equine synovial fluid. (Top) The stable diameter indicates that complexation-loaded TM1 remains sequestered in DMEM+10%FBS and in equine synovial fluid (ESF). (Bottom) The images showed TM1-loaded microgel-modified glass surfaces after exposure to DMEM + 10% FBS after 20h (left); and in ESF after 15h (right). The scale bar corresponds to 10 µm. As indicated by no change in microgel diameter, TM1 remains sequestered within PAA microgels when exposed to DMEM or to equine synovial fluid.

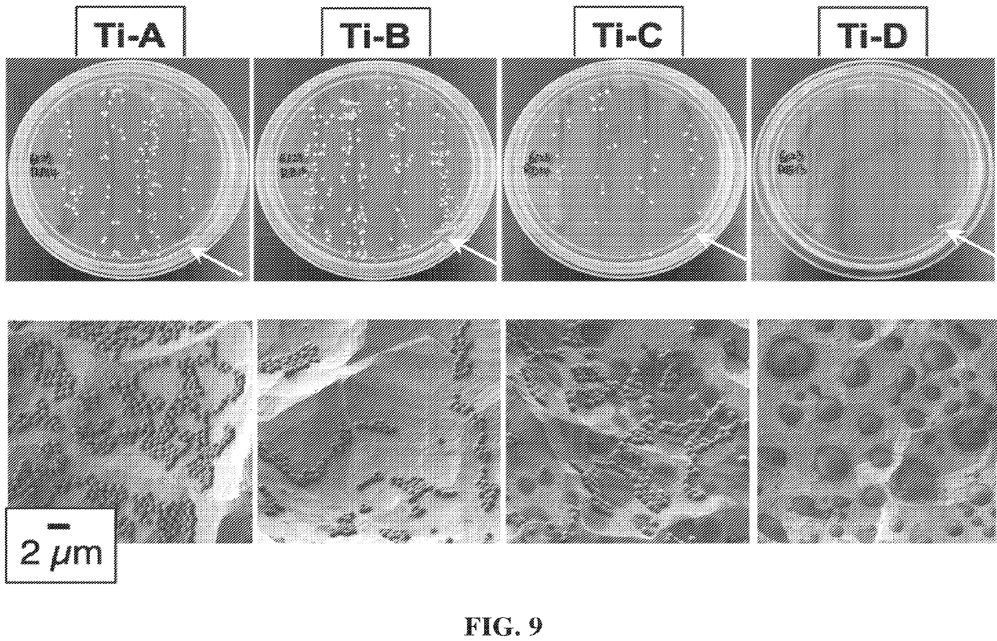

Fig. 9 is a series of images of TSA plates (24 h of culture) after rolling vortexed MSSA-contaminated Ti rods (white arrows). (Bottom) SEM images of the contaminated rods after rolling and culture. These images demonstrate results of the MSSA colonization assay for all four (A-D) surface modifications.
Fig. 10 is a series of images of TSA plates (24 h of culture) after rolling vortexed S. epidermidis-contaminated Ti rods (white arrows). (Bottom) SEM images of the contaminated rods after rolling and culture. These images demonstrate results of the S. epidermidis contamination assay for all four (A-D) surface modifications.

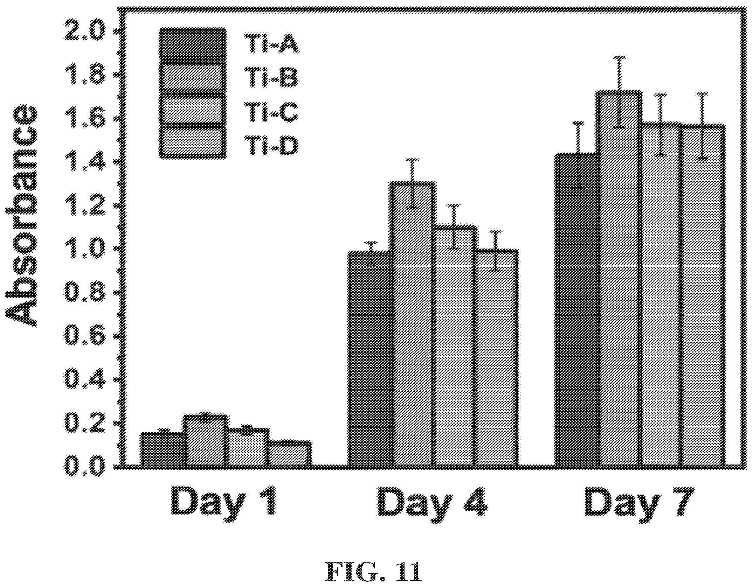

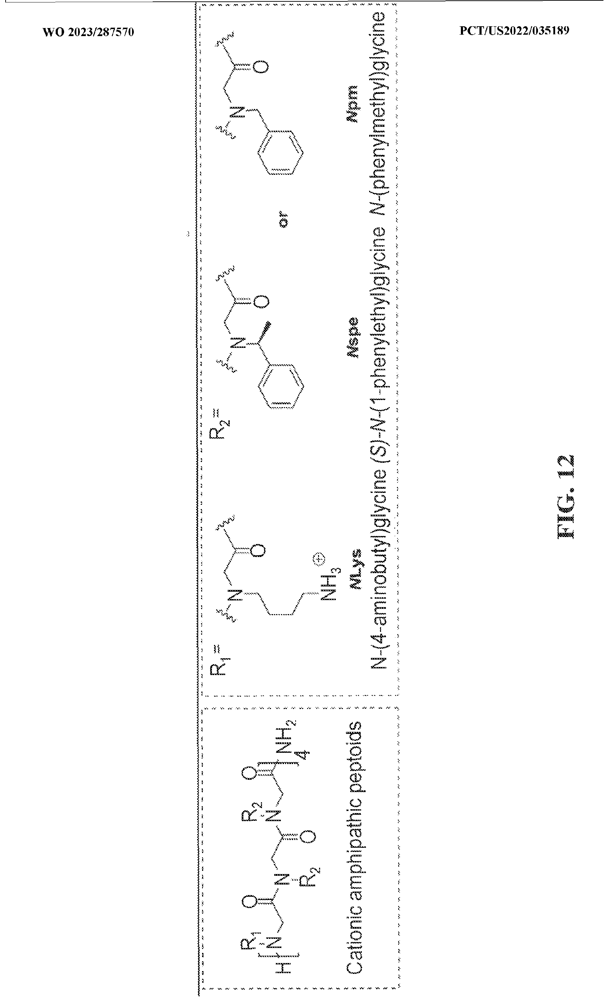

Fig. 11 is an MTS assay of hFOB metabolic activity on the four modified Ti surfaces. The assay characterizes metabolic activity of hFOB cells on the four (A-D) surface modifications.

### DETAILED DESCRIPTION

Self-defensive surfaces [14] represent an emerging strategy that may be able to inhibit bacterial colonization due to OR contamination. The term "self-defensive" was introduced by Boulmedais et al. [15] for the case of bacteria-triggered antimicrobial release. The mechanism at play with self-defensive surfaces is fundamentally different from conventional methods for inhibiting bacterial colonization through elutive drug delivery. In elutive techniques, antimicrobial compositions are continuously released from a surface whether they are needed or not. In contrast, a self-defensive mechanism releases the antimicrobial composition only when and where it is needed, and only in localized, very small amounts. Significantly, in the absence of a microbial challenge, self-defensive surfaces do not release antimicrobial compositions at all.

Self-defensive surfaces have been developed by Sukhishvili et al., [16] who designed polyelectrolyte thin-film coatings to release certain antimicrobial compositions in response to local pH changes. Cado, Boulmedais et al. [15a] exploited microbial enzyme secretion, and designed coatings that incorporate substrates for those particular enzymes, so that local coating degradation would release an antimicrobial composition. Both approaches are reliant on bacterial metabolism in order to become active. However, prior to implantation, a contaminated device surface has no source of nutrients, and thus, the occurrence of significant bacterial metabolism is unlikely. A self-defensive approach that relies on metabolism may thus not be appropriate for addressing OR contamination.

An alternate self-defensive mechanism, referred to as "contact transfer" [14c], has been developed and may be better suited to the problem of OR contamination. It has been shown [17] that contact transfer responds to the presence of challenging bacteria even in the absence of bacterial metabolism. In so doing, contact transfer drives the responsive release of certain antimicrobial compositions from a polyelectrolyte microgel coating, and can transfer those antimicrobial compositions to the challenging bacterium. This transfer has been attributed to the presence of a high concentration of negative charge on the bacterial envelope and the hydrophobicity of that envelop, which results in the complexation strength between the bacterium and the antimicrobial composition being greater than that between the microgel and the antimicrobial composition. Bacterial proximity to the loaded microgel then creates a steep chemical-potential gradient to drive antimicrobial de-complexation from the microgel and its transfer to the bacterium.

It has now been found that antimicrobial peptoids may be utilized in certain contact transfer surfaces to create effective self-defensive synthetic surfaces. Such surfaces may be fabricated, for example, by electrostatically depositing microgels of poly(acrylic acid) (PAA) to form a sub-monolayer coating on, for example, glass cover slips or titanium rods. In a subsequent self-assembly step, the sub-monolayer coating may be loaded with a suitable cationic antimicrobial peptoid. These self-defensive surfaces may significantly inhibit bacterial colonization, even in the absence of nutrients for metabolism. As described herein, the efficacy of these surfaces has been verified by testing in an in vitro model of OR contamination.

Various peptoids may be utilized in the self-defensive surfaces disclosed herein, although the use of the peptoid referred to as TM1 (also known as peptoid 1) is preferred.[18] Peptoids are analogs of peptides in which the side-groups are located on the amide nitrogen rather than on the α-carbon. In general, they are not proteolyzed,[19] thus improving their biostability and reducing immunogenicity. [19a] TM1 is a helical and amphipathic 12-mer peptoid consisting of a trimer (*N*Lys-*N*spe-*N*spe) repeated 4 times and terminated with a secondary amine group (see FIG. 1). Each of the four *N*Lys moieties includes a primary amine group. These five amine groups are all protonated under physiological conditions, and TM1 thus has a net electrostatic charge of +5. In addition, each *N*spe contains an aromatic moiety, which can contribute to complexation interactions. [20].

In situ optical microscopy may be utilized to track the peptoid loading process by imaging the microgel deswelling during complexation with the peptoid. Such imaging can also assess the ability of the peptoid to remain sequestered within the PAA microgels when exposed to physiologically relevant media. A digitally controlled aerosolizing system may be utilized to spray well-defined quantities of bacteria such as staphylococci (either S. aureus or S. epidermidis) onto Ti rods. Subsequent in vitro assays of bacterial viability and osteoblast response show that the peptoid-loaded microgel-modified surfaces inhibit bacterial colonization very effectively while still preserving cytocompatability comparable to unmodified controls.

### Materials and Methods

### Microgel synthesis, loading, and sequestration

Poly (acrylic acid) (PAA) microgels were synthesized by thermally initiated membrane emulsification. A precursor solution was mixed using 1.0 ml acrylic acid (Sigma), 0.47 g sodium hydroxide (NaOH, Sigma), 4 ml deionized (DI) water (Millipore type 1), 100 mg ammonium phosphate sulfate (APS, Sigma) and 100 µl Poly(ethylene glycol) diacrylate (PEGDA, Mₙ = 575 Da). This aqueous solution was forced via N₂ pressure (40 kPa) through a ceramic membrane (1.5 µm pore size (Shirasu Porous Glass (SPG)) into a stirred (400 rpm) oil phase consisting of 2.56 g Span 80 and 160 ml paraffin oil. The resulting emulsion was then deoxygenated for 30 min by N2 bubbling followed by heating to 70 °C and held there for 4 h under continuous stirring (500 rpm). After cooling to room temperature, the paraffin oil was removed by centrifugation and re-suspension first in cyclohexane two times, then in ethanol ten times, and finally in DI water ten times. The resulting PAA microgels were suspended in sterilized DI water and stored at 4 °C.

For in situ studies of microgel/antimicrobial interactions, a polydimethylsiloxane (PDMS) gasket was used to define 12 individual reaction chambers above a glass microscope slide. 6 mm diameter holes were punched from a cast sheet of cured PDMS (~4 mm thickness). The punched gasket was then pressed onto a pre-cleaned (3 min oxygen plasma) glass slide and annealed at 70 °C for 20 min. The volume of each glass-bottomed chamber was ~100 µl. The glass surface within each chamber was primed with positively charged poly(allylamine hydrochloride) (PAH, Sigma, Mw = 17.5 kDa) using an aqueous solution of 0.2 mg PAH/ml for 1 h followed by gentle washing using DI water and then drying by flowing N₂ gas. A sub-monolayer of PAA microgels was then electrostatically deposited onto the primed glass surface by filling each chamber with a colloidal aqueous microgel suspension at room temperature and left to soak for 30 min. These chambers were then rinsed with DI water and filled with 0.01 M phosphate buffer (pH 7.4, 4 mM NaH₂PO₄ and 6 mM Na₂HPO₄), [Na+]=0.016 M) prior to subsequent antimicrobial loading.

TM1 (M_{w} = 1819 Da) was synthesized as previously described.[21] It was loaded by complexation within the microgels by replacing the buffer within a particular reaction chamber with a solution of 1 mg TM1/ml of 0.01 M phosphate buffer. The loading process was followed by in situ time-resolved optical microscopy using an inverted microscope (Nikon, Eclipse Ti-E) equipped with a 14 bit CCD Camera (pco.pixelfly) and a CFI S Fluor ELWD 20× objective lens (NA = 0.45, WD = 8.2-6.9 mm). During the first 10 minutes of loading images were collected at one-minute intervals and then at five-minute intervals thereafter. After data acquisition, the time-resolved diameters of at least five different microgels were measured using ImageJ.[22] Each diameter was normalized using the initial diameter measured in TM1-free 0.01 M phosphate buffer.

The sequestration of the TM1 complexed within the microgels was again followed by in situ imaging. After loading, the TM1 solution was removed by gentle washing using DI water (3 times). Each reaction chamber was then filled with either autoclaved 0.01 M phosphate buffer or autoclaved PBS (phosphate buffered saline, pH 7.4, ionic strength = 0.138 M). The buffer was replaced with fresh buffer daily for 28 days. Optical micrographs of the same set of microgels were collected daily, and digital image analysis was used to measure the microgel diameter as a function of soaking time from at least five different microgels.

The amount of TM1 loaded into the microgels was quantified by UV absorption. Two aliquots of 10 µl of aqueous PAA microgel suspensions were prepared. One was allowed to fully dehydrate to measure the dry microgel weight. The other was added to 1 ml 0.01 M phosphate buffer containing 0.25 mg of TM1. After 60 min the loaded microgels were pelletized by centrifugation, and the UV adsorption (230 nm) of the supernatant was measured using a Synergy HT BioTek Spectrometer. The amount of loaded TM1 was then quantified by comparison with a calibration curve created using solutions of known TM1 concentrations in 0.01M phosphate buffer. The microgel zeta potential was measured using a Malvern ZETA SIZER Nano series from aliqouts of microgels suspended in pure 0.01 M phosphate buffer and before/after TM1 loading.

### OR Contamination Model

Commercially pure, surgical grade 1, titanium rods (1 cm length) were cut from wire (Temco RW0469) with an average diameter of 1.29 mm. The rods were rinsed twice with 70 % ethanol, sonicated in ethanol (15 min), washed and sonicated in autoclaved DI water, and finally dried using flowing N₂ gas. The dried rods were then exposed to an oxygen plasma for 10 min. Further modifications were made to create four surfaces: (Ti-A) plasma treated but otherwise unmodified Ti; (Ti-B) PAR-primed Ti; (Ti-C) microgel-modified (unloaded) Ti; and (Ti-D) TM1-loaded microgel-modified Ti. The PAH priming, electrostatic PAA microgel deposition, and the TM1 loading all followed the procedures described above for the glass substrates. The surfaces of each condition were imaged by scanning electron microcopy (SEM; Zeiss Auriga). TM1 loading for condition D was confirmed by exposing the modified pins to an aqueous solution of fluorescein isothiocyanate (FITC, Sigma) for 30 min in sterile DI water followed by repeated washing with DI water. The FITC-stained Ti-D rods were then imaged while hydrated using the Nikon Eclipse Ti-E inverted microscope with a C3 confocal attachment.

A digitally controlled aerosolizing system was used to contaminate the surfaces of rods in each of the four surface conditions with well-controlled quantities of bacteria. Details of the system configuration, operation, and properties have been described elsewhere.[23] The system is able to reproducibly spray well-defined bursts of aerosolized bacteria suspended in nutrient-free buffer onto test surfaces. Depending on the spray conditions, the density of sprayed bacteria is on the order of 10² - 10³/cm² and can thus mimic the contamination conditions of a surgical theater. The experiments here used methicillin-sensitive Staphylococcus aureus (MSSA, ATCC 29213) and Staphylococcus epidermidis (ATCC 35984). For each set of experiments, a single bacterial colony was inoculated into 10 ml of Tryptic Soy Broth (TSB) and grown to stationary phase at 37 °C under gentle shaking for 18 h. The bacteria were pelleted by centrifugation and resuspended in sterile PBS twice to remove the TSB. After final pelleting, the bacteria were suspended in sterile PBS to an optical density (OD600) of 0.0005A for MSSA and 0.0004A for S. epidermidis. These densities have been shown to produce contamination levels of about 10² CFU/cm².[23] The solutions were kept at room temperature and used within 3 hours of preparation. It has been shown [23] that over 90% of the bacteria remain culturable under these preparation conditions.

Two sets of three Ti rods from each of the four surface-modification conditions were sprayed with 100 ms bursts of bacterial aerosol. One set was sprayed with MSSA, and the other was sprayed with S. epidermidis. The three rods from each set were sprayed simultaneously. After spraying, the samples were left to dry in air within a laminar-flow biosafety hood for 30 min. The extent of contamination was then quantified in terms of the numbers of: (i) loosely bound culturable bacteria and (ii) strongly adhered culturable bacteria. Loosely bound bacteria were recovered by immersing each rod in 1.25 ml of sterile PBS followed by gentle vortexing. Three 250 µl aliquots of the buffer were then spread on separate tryptic soy agar (TSA) plates. Strongly adhered bacteria were assayed by gently washing each vortexed Ti rod using sterile PBS and then rolling each rod using flame-sterilized forceps over the surface of a TSA plate. Both sets of TSA plates were then cultured at 37 °C for 18 h. The number of colony-forming units (CFUs) was counted manually. The statistical significance of differences in bacterial colonization of the different surface-modification treatments was determined using a two-sided student t-test assuming unequal variances for the treated and control rods.

### Cytocompatibility

Human fetal osteoblasts (hFOB, ATCC, VA) were cultured in a 1:1 mixture of Dulbecco's Modified Eagle's Medium (DMEM) and Ham's F12 Medium, containing 10% fetal bovine serum (FBS, Atlantic Biologicals) and 2.5 mM L-glutamine (without phenol red). Prior to use, the culture was maintained at 34 °C with 5% CO₂ and 95% humidity until 70-80% confluence. To achieve a homogeneous cell distribution on individual substrates (Ti rods or glass cover slips) and equally among multiple substrates, hFOB cells were seeded and cultured following a specific protocol. Briefly, the Ti rods and coverslips sampling each of the four surface-modification conditions (A-D) were sterilized using 70% ethanol and then washed for 2 min with sterile DI water (3x). All samples were again sterilized under UV irradiation for 20 min before cell seeding. Each rod was first seeded with 100 µl of hFOB cell suspension, and the cell-seeded rod was then incubated for 15 min (34 °C, 5% CO₂, and 95% humidity) to promote cell attachment. The seeded rod was then flipped over and exposed again to 100 µl of hFOB cell suspension to achieve a final seeding density of 2 × 10⁴ cells/cm². Coverslips were seeded with 200 µl of hFOB cell suspension to a seeding density of 10⁴ cells/cm². After 30 min of total incubation, all cell-seeded samples were transferred into a new 6-well plate for further cultivation. The culture medium was refreshed every 2 days.

The adhesion (1 day) and proliferation (4 day and 7 day) of hFOB cells on the rods were assessed using an MTS assay. Briefly, after 1, 4, and 7 days, the culture rods (n=3 for each condition) were harvested, placed in a 12-well plate, and gently rinsed with cold PBS twice. Then, 1.4 mL of a mixture containing 400 µL MTS reagent (the assay kit was purchased from Promega, Madison, WI) and 1 mL culture medium was added each well. The plate was incubated for 2 h at 37 °C in 5% CO₂. 200 µL of supernatant from each sample was transferred to a 96-well plate. The absorbance at 490 nm was recorded using the BioTek Synergy microplate reader (BioTek Instruments, Inc., Vermont, USA). Statistically significant differences were assessed using a one-way t-test for the case where the Ti-A surface was used as the control and using an ANOVA test by Tukey's mean comparison (p < 0.05) for the case where the Ti-A, B and C surfaces were used as controls.

The cell morphologies on Ti wires and coverslips were characterized after cultivation of 1, 4, and 7 days. The specimens were fixed with 4% (w/v) paraformaldehyde for 15 min and washed for 2 min with PBS (3 times). Then, the samples were rinsed 3 times with 0.5% Triton X-100 and blocked with 3% (w/v) BSA to permeabilize the cell membranes. The cell nuclei were stained with 4', 6-diamidino-2-phenylindole (DAPI, Sigma, USA), and the cell skeletal filament actin (F-actin) was stained with Alexa Fluor 488-conjugated phalloidin. After staining, the samples were immersed in PBS and imaged while hydrated using a Nikon eclipse 80i epifluorescence microscope (Japan) and Zeiss LSM 880 confocal laser scanning microscope (Germany).

### Results and Discussion

When hydrated in 0.01 M phosphate buffer at pH 7.4 and [Na+] = 0.016 M, the as-synthesized PAA microgels have an average diameter of 6 ± 2 µm (Fig. S1). It has been shown previously [20] that the carboxyl groups in the PAA are fully deprotonated under these conditions. They can thus complex with oppositely charged macro-ions such as the TM1 peptoid studied here. The fact that TM1 can be loaded into the PAA microgels by complexation is shown by Fig. 2A. This plot follows the average microgel diameter during exposure to 0.01 M phosphate buffer with 1 mg/ml of TM1. The data are normalized to the unloaded diameter (6.0 µm) at time t=0 measured in TM1-free 0.01 M phosphate buffer. After 10 min, the normalized diameter decreases to ~55%. In a separate experiment, measuring the decrease in TM1 concentration from the loading solution by UV absorption shows that 39.6 µg of dry microgel loads 206 µg of TM1. These values indicate a ratio of 4.8 acrylic acid groups for each molecule of TM1, which is very close to the stoichiometry of five -1 acid groups for each +5 TM1 molecule. The microgel zeta potential furthermore increased from a value of -33.5 ± 4.5 mV (n=6) in the unloaded state (t=0) to 3.6 ± 1.2 mV (n=6) in the loaded state (both measurements made in 0.01 M phosphate buffer), again indicating charge neutralization due to TM1/PAA complexation.

Since device colonization is possible not only prior to implantation when the device is dry but also prior to surgical-site closure, an important question centers on whether the TM1 remains stably complexed within the PAA microgels when the device is in contact with physiological fluids. At a constant pH of 7.4, when the ionic strength is increased from [Na+] = 0.016 M in the 0.01 M phosphate loading buffer to [Na+] = 0.14 M typical of physiological conditions, the additional salt can shield the electrostatic pairing between amine groups on the TM1 and the acid groups within the PAA microgels. Such shielding, for example, leads to the burst release of colistin from PAA microgels.[17, 20] The fact that Fig. 2B shows no microgel diameter change when the loaded microgels are exposed to TM1-free PBS indicates stable TM1/PAA complexation. Similar experiments using flowing buffer with increasing concentrations of added NaCl indicate that a threshold [Na+] of about 0.35 M at pH 7.4 is needed to enable rapid release of the complexed TM1. This enhanced resistance to salting out in TM1/PAA relative to colistin/PAA is consistent with our recent observations that aromaticity enhances the complexation strength.[20] Exposure of TM1-loaded PAA microgels to serum-containing DMEM or to equine synovial fluid also does not induce changes in loaded microgel diameter and again indicates stable complexation in these more heterogeneous media (Fig. S2). The inset to Fig. 2B furthermore indicates stable TM1/PAA complexation in PBS for periods as long as four weeks.

Having developed a means to create TM1-loaded PAA-microgel modified surfaces by directed self-assembly, the contamination resistance of Ti rods subjected to each of four different surface treatments was investigated. These are illustrated schematically in the top panel of FIG. 3. The SEM images in the FIG. 3 bottom panel show the surface of rods modified with unloaded microgels (Ti-C) and with TM1-loaded microgels (Ti-D). The microgels form a sub-monolayer coating on the rough Ti surface with an inter-microgel spacing on the order of a few microns. The PAH-primed Ti surface is exposed between the microgels. The hydrated (unloaded) microgels comprise about 97% water [20]. So, when dried for SEM imaging, they flatten and exhibit little topography. In contrast, the TM1-loaded microgels have substantially more mass and less hydration, and they exhibit very distinctive topography when dried.

To mimic intraoperative contamination by airborne bacteria, three Ti rods were sprayed, sampling each of the four surface conditions with aerosols of 0.0067 M phosphate buffer (1× PBS with 0.0067 M PO4, pH 7.0 to 7.2) containing either MSSA or S. epidermidis. After spraying, the rods were left to dry and then assayed to determine the number of culturable bacteria either loosely bound or strongly adhered to the Ti surface. These two cases are differentiated mechanically by vortexing each rod in 0.0067 M phosphate buffer. Loosely bound bacteria were recovered in the buffer, and strongly adhered bacteria were recovered after vortexing by rolling the contaminated rod on an agar plate.

FIG. 4 summarizes the results of these experiments. The images compare the TM1-loaded microgel-modified Ti-D to the unmodified control Ti-A for the case of MSSA contamination. The results are strikingly different. MSSA colonies (white dots) are evident in each of the four rolling paths for the Ti-A while none can be see for the fully modified Ti-D. Recognizing that not all culturable bacteria are necessarily transferred from the rod (white arrows in the top images) to the agar, the rod surfaces were imaged via SEM. The lower left images of FIG. 4 show extensive colonization of the unmodified Ti-A. However, no bacteria were found by SEM imaging of the Ti-D surface, though the microgels clearly remain present. The fact that the microgels remain loaded is reflected by their topography. The graph in FIG. 4 quantifies the amount of loosely bound (solid blue) and strongly adhered (solid orange) MSSA for each of the four surface treatments. The crosshatched data show the results of similar experiments using S. epidermidis. The trends are very similar. The PAH-primed rods (Ti-B) exhibit the highest degree of contamination, which is consistent with the fact that the PAH is positively charged at pH 7.4 [24] and should attract bacteria, such as staphylococci, whose surface is negatively charged. Relative to the unmodified Ti-A, the (unloaded) microgel-modified Ti-C shows slightly fewer viable bacteria. This finding can be attributed to the fact that the microgels are less susceptible to colonization than the PAH-primed Ti surface, and the microgels block a fraction of the Ti surface.

Importantly, the number of culturable bacteria found on the TM1-loaded microgel-modified Ti-D samples is very small. In three trials, an average of only 2 CFUs of MSSA and only 1 CFU of S. epidermidis were found. These few CFUs were all recovered from the buffer after vortexing the contaminated rod and thus correspond to loosely bound colonies. No CFUs were found from bacteria strongly adhered to the Ti-D rods.

Since the three control surfaces all become significantly colonized, it is assumed that the Ti-D rods gare similarly exposed to culturable bacteria as a result of the spray-contamination process. The fact that only a very small number of colonies is subsequently found on the Ti-D rods indicates that the majority of these bacteria were killed by interaction with the surface. Neither the surface nor the medium (0.067 M phosphate buffer) in which the bacteria were suspended during the aerosolizing process provide nutrients, so it is expected that the bacteria will undergo little or no metabolism on the surface. Hence, triggering TM1 release from the loaded microgels via local pH reduction [25] is unlikely. Instead, these findings support the concept of contact transfer where the local chemical potential of the TM1 is lower in the bacterial envelope than it is within the microgels, [14c, 17] so that, when a bacterium comes within close proximity, some TM1 de-complexes from the microgel and re-complexes within the staphylococcal cell envelope. Such complexation interactions with bacteria have been extensively studied for a number of host-defense peptides and are attributed to the rich high concentration of anionic, hydrophobic, and aromatic moieties in the outer bacterial membranes. [26]

The fact that microgels, even when loaded, remain hydrophilic promotes opportunities for microgel-bacterium contact. If an aerosolized droplet impinges on a dry microgel-modified surface, water from that droplet will locally hydrate one or more microgels. As the overall droplet evaporates, the microgel(s) will be the most hydrophilic element of the surface and will be last to fully dry. Without wishing to be bound by theory, it is believed that surfaces forces associated with the edge of the drying droplet will draw any bacteria it may contain to a microgel, and when droplet evaporation is complete those bacteria will be concentrated on or near a microgel.

It is also notable that the in vitro model of OR contamination does not overwhelm the surface with bacteria as can be the case with in vivo infection models where a concentrated inoculum (e.g. 10⁶ - 10⁸ CFU/mL) is typically required in order to provoke infection in control animals such as rats and mice. The OR contamination model sprays low numbers of bacteria onto a rod surface. The probability of any particular microgel interacting with a bacterium is low. For that (much larger) subset of microgels that are unchallenged, the antimicrobial remains sequestered within these microgels (see the SEM image of the ToD rod in FIG. 4) and is available to face a subsequent bacterial challenge should one occur. That much smaller subset of microgels that do interact with a bacterium present a high local concentration of antimicrobial, which the assays of FIG. 4 indicate is sufficient to prevent surface colonization. The overall low number of bacteria involved in the contamination model again creates a situation where the probability of a second droplet landing on a TM1-depleted or partially depleted microgel is very low. Hence, microgel reloading, while in principle possible, is unnecessary.

Cell morphology (imaging) and metabolic activity (MTS) were utilized to evaluate the short-term cytocompatibility of the various surfaces. In the case of microgel-modified surfaces, only a fraction of the surface is covered by microgels. The underlying PAH-primed Ti surface is exposed between adjacent microgels and, because the PAH is cationic, it is expected that this exposed surface becomes covered by negatively charged serum proteins (e.g. albumin, fibronectin, etc.) when exposed to serum-containing medium. It has been previously shown that such microgel-modified surfaces remain highly compatible with both osteoblasts and macrophages.[17, 27] It is notable that the unmodified Ti surface has an intrinsic roughness over lateral length scales on the order of 2-10 µm, and that this roughness is further perturbed by the addition of TM1-loaded microgels with characteristic dimensions on the order of several microns. Such surface topography has long been known to affect and, in many cases, enhance cell adhesion, spreading, and proliferation.[28]

FIG. 5 shows the results of our cytocompatibility assays using human fetal osteoblasts. The cell metabolic activity was analyzed with MTS assay (FIG. 5 top). The absorbance for each sample set is normalized to its value of day 1 in order to better assess the proliferation rate independent of the initial cell adhesion. The raw MTS absorption data are presented in FIG. S5. Importantly, the metabolic activity measured from the fully modfied Ti-D samples is higher than that of the unmodified control samples (Ti-A). This indicates that the modified surface promotes the proliferation of hFOB cells. The imaging data in the bottom portion of FIG. 5, while qualitative, indicate that the hFOB cells adhere to, spread, and proliferate on the various rods in a similar fashion. These results show that the TM1-loaded microgels are not only effective in inhibiting bacterial colonization but also do not cause noticeable cell incompatibilities, at least as observed with these short-term in vitro assays.

Glass substrates were utilized to avoid many of the imaging complications associated with the curved and rough surface of the Ti rods. The top images in FIG. 6 show that the morphologies of hFOB cells cultured for 7 days on unmodified glass and on TM1-loaded microgel-modified glass are comparable. The top right image also shows significant green contrast (see white arrows) distributed across the surface. This fluorescence is observed from TM1-loaded microgels only when the samples were dried after loading and later rehydrated for subsequent culturing experiments. This contrast to TM1 self-assembly within the microgels may be attributed to hydrophobic interactions, hydrogen bonds, and π-π stacking, which has recently been documented when TM1 self-assembles into helical bundles.[29] Importantly, the modified Ti rods (Ti-D) were processed identically (loaded, dried, and later rehydrated), which indicates that antimicrobial properties are preserved even if the TM1 forms such aggregated bundles. The auto-fluorescence not only indicates the location of the microgels but further indicates that the TM1 remains loaded within the microgels despite the presence of the hFOB cells. This finding is consistent with previous results showing that culturing hFOB cells on PAA microgels loaded with the Sub5 antimicrobial peptide does not trigger AMP release.[17] SEM imaging (FIG. 6 bottom) shows that the hFOB cells are able to grow right over the TM1-loaded microgels.

### Conclusion

Exposure to the OR atmosphere between the time when a medical device is removed from its sterile package and the time when the wound site is fully closed can lead to device contamination by bacteria sedimenting from the atmosphere. That process has been modeled using an aerosolizing system able to spray small quantities of bacteria onto titanium rods. Even though relatively low numbers of bacteria contaminate each rod, when exposed to culture medium, bacteria sprayed onto unmodified Ti rods develop into proliferating colonies. The experiments described herein show that Ti surfaces modified with polyanionic microgels loaded by complexation with a cationic antimicrobial peptoid (preferably TM1) are able to almost entirely inhibit bacterial colony formation. Nutrients are unavailable to enable bacterial metabolism during the contamination process, indicating that the bacteria trigger local TM1 release by contact transfer rather than by local changes in pH. Because of the low numbers of contaminating bacteria, most of the loaded microgels are unchallenged during contamination, so their TM1 payload remains sequestered. However, neither the loaded peptoid nor the additional topography introduced by the microgels diminish the cytocompatibility of the modified surfaces as assayed by in vitro experiments with human fetal osteoblasts. This surface-modification strategy thus suggests a promising approach with which to inhibit the intra-operative bacterial colonization of exposed biomedical devices due to contamination in the operating theater.

While frequent reference has been made herein to TM1 (H-(NLys-Nspe-Nspe)₄-NH₂), various peptoids may be utilized in the devices, methodologies and compositions disclosed herein. Suitable peptoids may include, for example, the peptoids described in U.S.8,445,632 (Barron et al.), entitled "Selective Poly-N-Substituted Glycine Antibiotics", and the peptoids having the designations and structures depicted in TABLES 1-2 below.

**TABLE 1: Designations and Structures of Peptoids**

| **Peptoid** | **Structure** | **Chemical Formula** | **M.W.** |
|---|---|---|---|
| MXB001 | H-(*N*Lys-*N*spe-*N*spe)₄-NH₂ | C₁₀₄H₁₃₉N₁₇O₁₂ | 1819.36 |
| MXB002 | H-(*N*Lys-*N*spe-*N*spe(p-Br))₂-NH₂ | C₅₂H₆₉N₉O₆ | 1075.99 |
| MXB003 | H- *N*Lys-*N*spe-*N*spe-*N*Lys-*N*spe-*N*spe(p-Br)-NH₂ | | |
| MXB004 | H-((*N*Lys-*N*spe(p-Br)-*N*spe(p-Br))₂-NH₂ | C₅₂H₆₇Br₄N₉O₆ | 1233.78 |
| MXB005 | H-*N*tridec-*N*Lys-*N*spe-*N*spe-*N*Lys-NH₂ | C₄₇H₇₈N₈O₅ | 835.19 |
| MXB007 | H-(*N*Lys-*N*spe-*N*spe)₃-*N*Lys-*N*spe-NH₂ | | I |
| MXB008 | H-(*N*Lys-*N*spe-*N*spe)₂-NH₂ | C₆₄H₉₄N₁₀O₇ | 1115.52 |
| MXB009 | H-*N*dec-(*N*Lys-*N*spe-*N*spe)₂-NH₂ | C₆₄H₉₂N₁₀O₇ | 1273.31 |
| MXB010 | H- *N*dec-(*N*Lys-*N*spe-*N*spe(p-Br))₂-NH₂ | | |
| MXB011 | H- *N*tridec-(*N*lys-*N*spe-*N*spe(p-Br))₂-NH₂ | C₁₁₀H₁₅₁N₁₉O₁₃ | 1947.54 |
| MXB012 | H-(*N*Lys-*N*spe-*N*spe)₄-*N*Lys-NH₂ | C₅₈H₈₁Br₂N₁₁O₇ | 1204.16 |
| MXB013 | H-(*N*Lys-*N*spe-*N*spe(p-Br))₂-*N*Lys-NH₂ | | |
| MXB014 | H-*N*Lys-*N*spe-*N*spe-*N*Lys-*N*spe-*N*spe(p-Br)-*N*Lys-NH₂ | C₅₈H₇₉Br₄N₁₁O₇ | 1361.96 |
| MXB015 | H-(*N*Lys-*N*spe(p-Br)-*N*spe(p-Br))₂-*N*Lys-*N*H₂ | C₅₃H₉₀N₁₀O₆, | 962.70 |
| MXB016 | H-*N*tridec-*N*Lys-*N*spe-*N*spe-*N*Lys-*N*Lys-*N*H₂ | | |
| MXB017 | H-(*N*Lys-*N*spe-*N*spe)₃-*N*Lys-*N*spe-*N*Lys-NH₂ | | |
| MXB018 | H-(*N*Lys-*N*spe-*N*spe)₂-*N*Lys-NH₂ | | |
| MXB019 | H-*N*dec-(*N*Lys-*N*spe-*N*spe)₂-*N*Lys-NH₂ | C₇₀H₁₀₄Br₂N₁₂O₈ | 1401.49 |
| MXB020 | H- *N*dec-(*N*Lys-*N*spe-*N*spe(p-Br))₂-*N*Lys-NH₂ | | 1440 |
| MXB021 | H- *N*tridec-(*N*Lys-*N*spe-*N*spe(p-Br))₂-*N*Lys-NH₂ | | |
| MXB022 | H-(*N*Lys-*N*ssb-*N*ssb)₄-NH₂ | | |

Various cyclic peptoids may also be utilized in the devices, methodologies and compositions disclosed herein including, but not limited to, the peptoids disclosed in U.S. 9,938,321 (Kirshenbaum et al.), U.S. 9,315,548 (Kirshenbaum et al.) and U.S.8,828,413 (Kirshenbaum et al.).

In some embodiments, the peptoids disclosed herein are a poly-N-substituted glycine compound of a formula 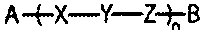 wherein
A is a terminal N-alkyl substituted glycine residue,
n is an integer,
B is selected from the group consisting of NH₂, one and two N-substituted glycine residues, and wherein said one and two N-substituted glycine residues have N-substituents which are independently selected from natural α-amino acid side chain moieties, isomers and carbon homologs thereof, and
X, Y and Z are independently selected from the group consisting of N-substituted glycine residues, wherein said N-substituents are independently selected from the group consisting of natural α-amino acid side chain moieties, isomers and carbon homologs thereof, and proline residues. In some embodiments, these peptoids may be utilized in combination with at least one other peptoid which is distinct from the first peptoid and which is a poly-N-substituted glycine compound of a formula 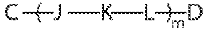 wherein
C is a terminal N-alkyl substituted glycine residue of the formula 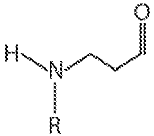
R is an alkyl group,
m is an integer,
D is selected from the group consisting of NH₂, one and two N-substituted glycine residues, and wherein said one and two N-substituted glycine residues have N-substituents which are independently selected from natural α-amino acid side chain moieties, isomers and carbon homologs thereof, and
J, K and L are independently selected from the group consisting of N-substituted glycine residues, wherein said N-substituents are independently selected from the group consisting of natural α-amino acid side chain moieties, isomers and carbon homologs thereof, and proline residues.

The peptoids in the compositions described herein may be alkylated, and preferably have terminal alkylation. Here, alkylation (and especially terminal alkylation) with a C10 or C13 tail is especially preferred. It has been found that such terminal alkylation may enhance the efficacy of the peptoid in some applications, and in some cases, may cause a peptoid which otherwise has low antibacterial activity to have significant antibacterial activity. Thus, in the foregoing formula, R may be selected from about C4 to about C20 linear, branched and cyclic alkyl moieties. R preferably contains at least 8 carbon atoms, more preferably contains at least 10 carbon atoms, and most preferably contains at least 12 carbon atoms. Embodiments where R is a decyl or tridecyl moiety are especially preferred.

It will further be appreciated that various salts or precursors of the foregoing peptoids may be utilized in the compositions and methodologies disclosed herein. As an example of the latter, these compositions and methodologies may contain one or more precursors of the general formula A-P, where P is one of the foregoing peptoids (or a salt thereof) and A is a moiety which acts as a leaving group to generate P when the precursor undergoes an in vivo reaction (such as, for example, proteolytic degradation).

Various halogenated peptoids and halogenated oligomers of N-substituted glycines (and salts thereof) may also be utilized in the compositions and methodologies disclosed herein. These include, without limitation, various halogenated analogs of the foregoing peptoids and oligomers of N-substituted glycines. Examples of such halogenated peptoids are described, for example, in WO2020223581A1 (Molchanova et al.), entitled "Halogenated Antimicrobial Peptoids". These halogenated compositions may be halogenated in various ways. For example, these compounds may include any number of halogen substitutions with the same or different halogens. In particular, these compounds may include one or more fluoro-, chloro-, bromo- or iodo- substitutions, and may include substiutution with two or more distinct halogens. However, the use of one or two bromo- or chloro-substitutions is preferred in many applications. Moreover, while the peptoids described herein may be halogenated at various locations, para halogenation on the peptoids containing aryl rings is especially preferred in many applications, although ortho- and meta- substitution, or even perhalogentation, may be useful in some applications.

Any of the peptoids disclosed in [Nam, H.Y., Choi, J., Kumar, S.D., Nielsen, J.E., Kyeong, M., Wang, S., Kang, D., Lee, Y., Lee, J., Yoon, M.-H., Hong, S., Lund, R., Jenssen, H., Shin, S.Y., Seo, J., 2020. Helicity Modulation Improves the Selectivity of Antimicrobial Peptoids. ACS Infectious Diseases 6, 2732-2744. doi:10.1021/acsinfecdis.0c00356] may also be utilized in the compositions and methodologies disclosed herein. These specifically include the peptoids depicted in TABLE 2 below.

**TABLE 2: Sequences and Properties of Select Peptoids**

| Sequence | Number of *N*spe |
|---|---|
| H-*N*Lys-*N*spe- *N*spe-*N*Lys-*N*spe-*N*spe-*N*spe-*N*spe-*N*spe-*N*Lys-*N*spe-*N*spe-NH₂ | 8 |
| H-*N*Lys-*N*pm- *N*pm-*N*Lys-*N*pm-*N*pm-*N*Lys-*N*pm-*N*pm-*N*Lys-*N*pm-*N*pm-NH₂ | 0 |
| H-*N*Lys-*N*spe- *N*pm-*N*Lys-*N*pm-*N*pm-*N*Lys-*N*pm-*N*pm-*N*Lys-*N*pm-*N*pm-NH₂ | 1 |
| H-*N*Lys-*N*pm- *N*spe-*N*Lys-*N*pm-*N*pm-*N*Lys-*N*pm-*N*pm-*N*Lys-*N*pm-*N*pm-NH₂ | 1 |
| H-*N*Lys-*N*pm- *N*pm-*N*Lys-*N*spe-*N*pm-*N*Lys-*N*pm-*N*pm-*N*Lys-*N*pm-*N*pm-NH₂ | 1 |
| H-*N*tys-*N*pm- *N*pm-*N*Lys-*N*pm-*N*spe-*N*tys-*N*pm-*N*pm-*N*Lys-*N*pm-*N*pm-NH₂ | 1 |
| H-*N*Lys-*N*pm- *N*pm-*N*Lys-*N*pm-*N*pm-*N*Lys-*N*spe-*N*pm-*N*Lys-*N*pm-*N*pm-NH₂ | 1 |
| H-*N*Lys-*N*pm- *N*pm-*N*Lys-*N*pm-*N*pm-*N*Lys-*N*pm-*N*spe-*N*Lys-*N*pm-*N*pm-NH₂ | 1 |
| H-*N*Lys-*N*pm- *N*pm-*N*Lys-*N*pm-*N*pm-*N*Lys-*N*pm-*N*pm-*N*Lys-spe-*N*pm-NH₂ | 1 |
| H-*N*Lys-*N*pm- *N*pm-*N*Lys-*N*pm-*N*pm-*N*Lys-*N*pm-*N*pm-*N*Lys-*N*pm-*N*spe-NH₂ | 1 |
| H-*N*Lys-*N*spe- *N*pm-*N*Lys-*N*pm-*N*pm-*N*Lys-*N*pm-*N*pm-*N*Lys-*N*pm-*N*spe-NH₂ | 2 |
| H-*N*Lys-*N*spe- *N*spe-*N*Lys-*N*pm-*N*pm-*N*Lys-*N*pm-*N*pm-*N*Lys-*N*pm-*N*pm-NH₂ | 2 |
| H-*N*Lys-*N*spe- *N*spe-*N*Lys-*N*pm-*N*pm-*N*Lys-*N*pm-*N*pm-*N*Lys-*N*pm-*N*spe-NH₂ | 3 |
| H-*N*Lys-*N*spe- *N*pm-*N*Lys-*N*pm-*N*pm-*N*Lys-*N*pm-*N*pm-*N*Lys-*N*pm-*N*spe-NH₂ | 3 |
| H-*N*Lys-*N*pm- *N*pm-*N*Lys-*N*pm-*N*pm-*N*Lys-*N*pm-*N*pm-*N*Lys-*N*pm-*N*pm-NH₂ | 3 |
| H-*N*Lys-*N*spe- *N*spe-*N*Lys-*N*pm-*N*pm-*N*Lys-*N*pm-*N*pm-*N*Lys-*N*spe-*N*spe-NH₂ | 4 |
| H-*N*Lys-*N*pm-*N*pm-*N*Lys-*N*spe-*N*spe-*N*Lys-*N*spe-*N*spe-*N*Lys-*N*pm-*N*pm-NH₂ | 4 |

Various biomedical devices may be treated in accordance with the teachings herein. The biomedical device is one of a hip/knee prosthesis, a heart valve, a pacemaker, a cochlear implant, a shunt, a surgical mesh, a suture, or a tissue-engineering construct. The biomedical device may be of a type designed to be implanted in the body of a subject, which may be a human or animal subject.

Preferred embodiments of the methodologies disclosed herein involve treating a surface of a biomedical device by depositing a polyanionic microgel (or in some embodiments, a nanogel) onto the surface of the biomedical device and loading the deposited polyanionic microgel with a peptoid. In some embodiments, the surface of the biomedical device may be primed with a polycation layer prior to depositing the polyanionic microgel. The polyanionic microgel may be synthesized by a process which includes membrane emulsification and UV photopolymerization and is preferably deposited as a sub-monolayer coating on the surface of the biomedical device. Preferably, the polyanionic microgel comprises polyacrylic acid.

The surfaces of biomedical devices treated in accordance with the methodologies disclosed herein may comprise various materials. These include, without limitation, materials selected from the group consisting of metals, metal oxides, and metal alloys such as, for example, alumina, zirconia, and zirconia toughened alumina (ZTA); refractory metals, such as Zr, Ta, V, Nb, W, Mo and alloys thereof; metal alloys, such as, for example, Ti-6Al-4V alloys, Ti-5Al-2.5Fe alloys, Ti-6Al-7Nb alloys and β-Ti alloys (including Ti-12Mo-6Zr-2Fe (TMZF)); titanium alloys, such as, for example, Co-Cr-Mo alloys, stainless steel alloys, and refractory metal alloys; oxidized zirconium (oxinium); alumina, zirconia, and zirconia toughened alumina (ZTA); magnesia partially stabilized zirconia (MgPSZ); and yttria stabilizing oxide (Y-TZP). The surfaces of biomedical devices treated in accordance with the methodologies disclosed herein may also comprise various organic polymers such as, for example, polyethylene (including ultra-high-molecular-weight polyethylene (UHMWPE) and highly cross-linked polyethylene (HXLPE)), polytetrafluoroethylene (PTFE) or polyetheretherketone (PEEK).

The self-defensive surfaces disclosed herein may be adapted to release one or more peptoids in the presence of various pathogens, including various bacteria, fungi and viruses. Specific examples of such pathogens may include *Staphylococcus aureus, Pseudomonas aeruginosa, Escherichia coli, Klebsiella, Proteus, Enterobacter, Clostridium difficile,* and *Salmonela, Streptoccoci*; *Candida albicans, Aspergillus spp., Nocardia, Pneumocystis carinii, Cryptococcus neoformans, and Cryptosporidium;* respiratory syncytial virus, cytomegalovirus, human immunodeficiency virus (HIV), Ebola, rotavirus, enteroviruses, Influenza A (including subtypes H2N2 and H3N3), hepatitis and herpes viruses.

Various peptoid release surfaces may be created in accordance with the teachings herein. These release surfaces preferably comprise microgels, but in some embodiments may also comprise nanogels. The release surface preferably comprises a zwitterionic polymer, such as a polybetaine. Such a polymer may contain cationic groups such as, for example, quaternary ammonium groups; and anionic groups such as, for example, carboxylate, sulfonate, phosphate, phosphinate, and phosphonate groups.

In some embodiments, methods are provided for treating a surface of a biomedical implant to render it resistant to bacterial cultivation. In such embodiments, the method may comprise (a) priming a surface of the implant with poly(allylamine hydrochloride), thereby obtaining a primed surface; (b) applying a sub-monolayer of poly(acrylic acid) (PAA) microgels to the primed surface, thereby obtaining a 3D cross-linked colloidal structure; and (c) loading the colloidal structure with a peptoid by forming a complex between the PAA microgels and the peptoid. The poly(allylamine hydrochloride) is preferably positively charged, and treating a surface of the implant with poly(allylamine hydrochloride) may include (a) applying an aqueous solution of poly(allylamine hydrochloride) to the surface, thereby forming a treated surface; (b) rinsing the treated surface, thereby obtaining a rinsed surface; and (c) drying the rinsed surface. Applying a sub-monolayer of PAA microgels to the primed surface may include electrostatically depositing PAA onto the primed surface, which may include exposing the primed surface to a colloidal aqueous microgel suspension. Similarly, loading the PAA microgels with a peptoid may include exposing the PAA microgels to a buffered solution of the peptoid, in which case the use of a phosphate buffer is especially preferred. The loading process preferably includes forming a complex between the PAA microgels and the peptoid.

PAA microgels of various dimensions may be utilized in the constructs and methodologies described herein. Preferably, the PAA ls have an average diameter within the range of 6 ± 2 µm prior to loading the PAA microgels with a peptoid. Loading the PAA microgels with a peptoid preferably results in a ratio R_{aa/pep} of acrylic acid groups to peptoid within the range of 1 to 50, more preferably 2 to 20, even more preferably 3 to 10, and most preferably 4 to 5. The microgel zeta potential preferably increases from a value of -33.5 ± 4.5 mV in the unloaded state to 3.6 ± 1.2 mV in the loaded state.

The above description of the present invention is illustrative, and is not intended to be limiting. Accordingly, the scope of the present invention should be construed in reference to the appended claims.

### REFERENCES

[1] a)W. H. Organization, Global Gidelinesfor the Pevention of Surgical Site Infection, WHO, 2017**;** b)R. R. W. Brady, J. Verran, N. N. Damani, A. P. Gibb, J. Hosp. Infect. 2009, 71, 295; c)D. Chauveaux, Orthop. Traumatol.: Surg. Res. 2015, 101, S77; d)T. T. Chow, X. Y. Yang, J. Hosp. Infect. 2004, 56, 85; e)K. Goswami, K. L. Stevenson, J. Parvizi, J. Arthroplasty 2020, 35, S2; f)S. M. McHugh, A. D. K. Hill, H. Humphreys, Surgeon 2015, 13, 52; g)J. Parvizi, S. Barnes, N. Shohat, C. E. Edmiston, Jr., Am. J. Infect. Control 2017, 45, 1267; h)P. Weaving, F. Cox, S. Milton, J Perioper Pract 2008, 18, 199.
[2] R. H. Fitzgerald, Jr., Arch. Surg. 1979, 114, 772.
[3] a)M. Diab-Elschahawi, J. Berger, A. Blacky, O. Kimberger, R. Oguz, R. Kuelpmann, A. Kramer, O. Assadian, Am. J. Infect. Control 2011, 39, e25; b)C. Napoli, V. Marcotrigiano, M. T. Montagna, BMC Public Health 2012, 12, 594; c)C. Pasquarella, G. E. Sansebastiano, S. Ferretti, E. Saccani, M. Fanti, U. Moscato, G. Giannetti, S. Fornia, P. Cortellini, P. Vitali, C. Signorelli, J. Hosp. Infect. 2007, 66, 313.
[4] a)S. Kurtz, K. Ong, E. Lau, F. Mowat, M. Halpern, J. Bone Jt. Surg. Ser. A 2007, 89, 780; b)B. D. Springer, S. Cahue, C. D. Etkin, D. G. Lewallen, B. J. McGrory, Arthroplast. Today 2017, 3, 137; c)K. J. Bozic_(Committee Chair), American Academy of Orthopaedic Surgeons 2018*.*
[5] T. J. Cahill, B. D. Prendergast, The Lancet 2016, 387, 882.
[6] M. Döring, S. Richter, G. Hindricks, Dtsch. Arztebl. Inter. 2018, 115, 445.
[7] J. K.-L. Choong, S. J. O'Leary, in Infections of the Ears, Nose, Throat, and Sinuses, (Eds: M. L. Durand, D. G. Deschler), Springer International Publishing AG, part of Springer Nature, 2018.
[8] Y. Gutierrez-Murgas, J. N. Snowden, J. Neuroimmunol. 2014, 276, 1.
[9] M. Arnold, A. Kao, K. Gbozah, B. Heniford, V. Augenstein, International Journal of Abdominal Wall and Hernia Surgery 2018, 1, 42.
[10] I. Ahmed, A. J. Boulton, S. Rizvi, W. Carlos, E. Dickenson, N. A. Smith, M. Reed, BMJ Open 2019, 9, e29727.
[11] R. Kuijer, E. J. P. Jansen, P. J. Emans, S. K. Bulstra, J. Riesle, J. Pieper, D. W. Grainger, H. J. Busscher, Biomaterials 2007, 28, 5148.
[12] a)H. K. Anis, N. Sodhi, A. K. Klika, M. A. Mont, W. K. Barsoum, C. A. Higuera, R. M. Molloy, J. Arthroplasty 2019, 34, S331; b)H. Cheng, B. P. H. Chen, I. M. Soleas, N. C. Ferko, C. G. Cameron, P. Hinoul, Surg. Infect. 2017, 18, 722; c)H. Cheng, J. W. Clymer, B. Po-Han Chen, B. Sadeghirad PhD, N. C. Ferko, C. G. Cameron, P. Hinoul, J. Surg. Res. 2018, 229, 134; d)B. Ravi, R. Jenkinson, S. O'Heireamhoin, P. C. Austin, S. Aktar, T. S. Leroux, M. Paterson, D. A. Redelmeier, EClinicalMedicine 2019, 16, 74.
[13] a)A. Baldini, K. Blevins, D. Del Gaizo, O. Enke, K. Goswami, W. Griffin, P. F. Indelli, T. Jennison, E. Kenanidis, P. Manner, R. Patel, T. Puhto, P. Sancheti, R. Sharma, R. Sharma, R. Shetty, R. Sorial, N. Talati, T. D. Tarity, K. Tetsworth, C. Topalis, E. Tsiridis, A. W-Dahl, M. Wilson, J. Arthroplasty 2019, 34, S97; b)C. L. Barnes, A. M. Cooper, J. Luque, J. Manghwani, W. Y. Matar, I. Panda, A. Rajgopal, S. Vaidya, O. Wakde, J. Arthroplasty 2019, 34, S175; c)A. M. Cooper, A. J. Shope, M. Javid, A. Parsa, M. A. Chinoy, J. Parvizi, J. Bone Jt. Surg. Am. Vol. 2019, 101, e133; d)J. Parvizi, T. Gehrke, M. A. Mont, J. J. Callaghan, J. Arthroplasty 2019, 34, S1.
[14] a)W. Ahmed, Z. Zhai, C. Gao, Materials Today Bio 2019, 2; b)T. Wei, Q. Yu, H. Chen, Adv Healthc Mater 2019, 8, e1801381; c)X. Xiao, W. Zhao, J. Liang, K. Sauer, M. Libera, Colloids and Surfaces B: Biointerfaces 2020, 192, 110989.
[15] a)G. Cado, R. Aslam, L. Séon, T. Garnier, R. Fabre, A. Parat, A. Chassepot, J. C. Voegel, B. Senger, F. Schneider, Y. Frère, L. Jierry, P. Schaaf, H. Kerdjoudj, M. H. Metz-Boutigue, F. Boulmedais, Advanced Functional Materials 2013**;** b)L. Séon, P. Lavalle, P. Schaaf, F. Boulmedais, Langmuir 2015, 31, 12856.
[16] a)S. Pavlukhina, Y. Lu, A. Patimetha, M. Libera, S. Sukhishvili, Biomacromolecules 2010, 11, 3448; b)I. Zhuk, F. Jariwala, A. B. Attygalle, Y. Wu, M. Libera, S. A. Sukhishvili, ACS Nano 2014, 8, 7733.
[17] J. Liang, H. Wang, M. Libera, Biomaterials 2019, 204, 25.
[18] N. P. Chongsiriwatana, J. A. Patch, A. M. Czyzewski, M. T. Dohm, A. Ivankin, D. Gidalevitz, R. N. Zuckermann, A. E. Barron, P Natl Acad Sci USA 2008, 105, 2794.
[19] a)J. A. Gibbons, A. A. Hancock, C. R. Vitt, S. Knepper, S. A. Buckner, M. E. Brune, I. Milicic, J. F. Kerwin, L. S. Richter, E. W. Taylor, K. L. Spear, R. N. Zuckermann, D. C. Spellmeyer, R. A. Braeckman, W. H. Moos, J. Pharmacol. Exp. Ther. 1996, 277, 885; b)S. M. Miller, R. J. Simon, S. Ng, R. N. Zuckermann, J. M. Kerr, W. H. Moos, Drug Develop Res 1995, 35, 20; c)S. M. Miller, R. J. Simon, S. Ng, R. N. Zuckermann, J. M. Kerr, W. H. Moos, Drug Dev Res 1995, 35, 20.
[20] X. Xiao, J. Ji, W. Zhao, S. Nangia, M. Libera, Macromolecules 2022, 55, 1736.
[21] G. Diamond, N. Molchanova, C. Herlan, J. A. Fortkort, J. S. Lin, E. Figgins, N. Bopp, L. K. Ryan, D. Chung, R. S. Adcock, M. Sherman, A. E. Barron, Pharmaceuticals 2021, 14, 304.
[22] a)J. Schindelin, I. Arganda-Carreras, E. Frise, V. Kaynig, M. Longair, T. Pietzsch, S. Preibisch, C. Rueden, S. Saalfeld, B. Schmid, J.-Y. Tinevez, D. J. White, V. Hartenstein, K. Eliceiri, P. Tomancak, A. Cardona, Nature Methods 2012, 9, 676; b)C. A. Schneider, W. S. Rasband, K. W. Eliceiri, Nature Methods 2012, 9, 671.
[23] W. Zhao, M. Libera, M. Prysak, J. Katz, L. De Stefano, Journal of Biomedical Materials Research Part B: Applied Biomaterials 2022**,** submitted.
[24] J. Choi, M. F. Rubner, Macromolecules 2005, 38, 116.
[25] V. Albright, I. Zhuk, Y. Wang, V. Selin, B. van de Belt-Gritter, H. J. Busscher, H. C. van der Mei, S. A. Sukhishvili, Acta Biomater. 2017, 61, 66.
[26] a)N. Malanovic, K. Lohner, Biochim. Biophys. Acta Biomembr. 2016, 1858, 936; b)N. Mookherjee, M. A. Anderson, H. P. Haagsman, D. J. Davidson, Nat. Rev. Drug Discov. 2020, 19, 311; c)W. C. Wimley, ACS Chem. Biol. 2010, 5, 905.
[27] a)Q. Wang, M. Libera, Colloids and Surfaces B: Biointerfaces 2014, 118, 202; b)Y. Wang, G. Subbiahdoss, J. Swartjes, H. C. van der Mei, H. J. Busscher, M. Libera, Advanced Functional Materials 2011, 21, 3916.
[28] a)C. C. Berry, G. Campbell, A. Spadiccino, M. Robertson, A. S. G. Curtis, Biomaterials 2004, 25, 5781; b)S. A. Biela, Y. Su, J. P. Spatz, R. Kemkemer, Acta Biomater. 2009, 5, 2460; c)D. Hoffman-Kim, J. A. Mitchel, R. V. Bellamkonda, in Annual Review of Biomedical Engineering, Vol. 12, 2010; d)Y. Hu, J. O. You, J. Aizenberg, ACS Appl. Mater. Interfaces 2016, 8, 21939; e)M. Nikkhah, F. Edalat, S. Manoucheri, A. Khademhosseini, Biomaterials 2012, 33, 5230.
[29] J. E. Nielsen, M. A. Alford, D. B. Y. Yung, N. Molchanova, J. A. Fortkort, J. S. Lin, G. Diamond, R. E. W. Hancock, H. Jenssen, D. Pletzer, R. Lund, A. E. Barron, ACS Infect. Dis. 2021**.**

## Claims

1. A biomedical device, comprising:
a surface selected from the group consisting of metal surfaces, ceramic surfaces and polymeric surfaces;
a polyanionic microgel disposed on said surface; and
a peptoid disposed in said polyanionic microgel;
wherein the biomedical device is a hip prosthesis, a knee prosthesis, a heart valve, a pacemaker, a cochlear implant, a shunt, a surgical mesh, a suture or a tissue-engineering construct.

2. The biomedical device of claim 1, wherein said surface comprises:
at least one material selected from the group consisting of metals, metal oxides, metal alloys, and polymeric materials; or
at least one material selected from the group consisting of alumina, zirconia, and zirconia toughened alumina (ZTA); or
at least one refractory metal; or
at least one titanium alloy; or
stainless steel.

3. The biomedical device of claim 2, wherein said surface comprises polymeric material, and said polymeric material includes:
at least one material selected from the group consisting of polyethylene, polytetrafluoroethylene (PTFE) and polyetheretherketone (PEEK); or
at least one material selected from the group consisting of ultra-high-molecular-weight polyethylene (UHMWPE) and highly cross-linked polyethylene (HXLPE).

4. The biomedical device of claim 2_{,} wherein said surface comprises at least one metal alloy selected from the group consisting of Co-Cr-Mo alloys, stainless steel alloys

5. The biomedical device of claim 2, wherein said surface comprises refractory metal selected from the group consisting of Zr, Ta, V, Nb, W, Mo and alloys thereof.

6. The biomedical device of claim 5, wherein said surface comprises:
an alloy selected from the group consisting of Ti-6Al-4V alloys, Ti-5Al-2.5Fe alloys, Ti-6Al-7Nb alloys and β-Ti alloys; or
oxidized zirconium (oxinium).

7. The biomedical device of claim 6, wherein said surface comprises Ti-12Mo-6Zr-2Fe (TMZF).

8. The biomedical device of claim 2, wherein said surface comprises a material selected from:
the group consisting of alumina, zirconia, and zirconia toughened alumina; or
the group consisting of magnesia partially stabilized zirconia (MgPSZ) and yttria stabilizing oxide (Y-TZP).

9. The biomedical device of claim 1, wherein said peptoid is H-(NLys-Nspe-Nspe)₄-NH₂.

10. The biomedical device of claim 1, wherein said peptoid is selected from:
the group consisting of: H-(*N*Lys-*N*spe-*N*spe)₄-NH₂, H-(*N*Lys-*N*spe-*N*spe(p-Br))₂-NH₂, H-*N*Lys-*N*spe-*N*spe-*N*Lys-*N*spe-*N*spe(p-Br)-NH₂, *H*-((*N*Lys-*N*spe(p-Br)-*N*spe(p-Br))₂-NH₂, H*-N*tridec-*N*Lys-*N*spe-*N*spe-*N*Lys-*N*H₂, H-(*N*Lys-*N*spe-*N*spe)₃-*N*Lys-*N*spe-*N*H₂, H-(*N*Lys-*N*spe-*N*spe)₂-NH₂, H-*N*dec-(*N*Lys-*N*spe-*N*spe)₂-NH₂, H-*N*dec-(*N*Lys-*N*spe-*N*spe(p-Br))₂-NH₂, H- Ntridec-(*N*Lys-*N*spe-*N*spe(p-Br))₂-NH₂, H-(*N*Lys-*N*spe-*N*spe)₄-*N*Lys-NH₂, H-(*N*Lys-*N*spe-*N*spe(p-Br))₂-*N*Lys-*N*H₂, H-*N*Lys-*N*spe-*N*spe-*N*Lys-*N*spe-*N*spe(p-Br)-*N*Lys-NH₂, H-(*N*Lys-*N*spe(p-Br)-*N*spe(p-Br))₂-*N*Lys-*N*H₂, H-*N*tridec-*N*Lys-*N*spe-*N*spe-*N*Lys-*N*Lys-NH₂, H-(*N*Lys-*N*spe-*N*spe)₃-*N*Lys-*N*spe-*N*Lys-NH₂, H-(*N*Lys-*N*spe-*N*spe)₂-*N*Lys-NH₂, H-*N*dec-(*N*Lys-*N*spe-*N*spe)₂-*N*Lys-NH₂, H-*N*dec-(*N*Lys-*N*spe-*N*spe(p-Br))₂-*N*Lys-*N*H₂, H-*N*tridec-(*N*Lys-*N*spe-*N*spe(p-Br))₂-*N*Lys-*N*H₂, and H-(*N*Lys-*N*ssb-*N*ssb)₄-*N*H₂; or
the group consisting of: H-(*N*Lys-*N*spe-*N*spe(p-Br))₂-NH₂, H-*N*Lys-*N*spe-*N*spe-*N*Lys-*N*spe-*N*spe(p-Br)-NH₂, H-((*N*Lys-*N*spe(p-Br)-*N*spe(p-Br))₂-NH₂, H-*N*dec-(*N*Lys-*N*spe-*N*spe(p-Br))₂-NH₂, H- Ntridec-(*N*Lys-*N*spe-*N*spe(p-Br))₂-NH₂, H-(*N*Lys-*N*spe-*N*spe(p-Br))₂-*N*Lys-NH₂, H-*N*Lys-*N*spe-*N*spe-*N*Lys-*N*spe-*N*spe(p-Br)-*N*Lys-NH₂, H-(*N*Lys-*N*spe(p-Br)-*N*spe(p-Br))₂-*N*Lys-NH₂, H-*N*dec-(*N*Lys-*N*spe-*N*spe(p-Br))₂-*N*Lys-*N*H₂, and H-Ntridec-(*N*Lys-*N*spe-*N*spe(p-Br))₂-*N*Lys-*N*H₂; or
the group consisting of: H-NLys-Nspe- Nspe-NLys-Nspe-Nspe-NLys-Nspe-Nspe-NLys-Nspe-Nspe-NH₂, H-*N*Lys-*N*pm-*N*pm-*N*Lys-*N*pm-*N*pm-*N*Lys-*N*pm-*N*pm-*N*Lys-*N*pm-*N*pm-NH₂, H-*N*Lys-*N*spe- *N*pm-*N*Lys-*N*pm-*N*pm-*N*Lys-*N*pm-*N*pm-*N*Lys-*N*pm-*N*pm-NH₂, H-*N*Lys-*N*pm- *N*spe-*N*Lys-*N*pm-*N*pm-*N*Lys-*N*pm-*N*pm-*N*Lys-*N*pm-*N*pm-NH₂, H-*N*Lys-*N*pm-*N*pm-*N*Lys-*N*spe-*N*pm-*N*Lys-*N*pm-*N*pm-*N*Lys-*N*pm-*N*pm-NH₂, H-*N*Lys-*N*pm- Npm-NLys-*N*pm-*N*spe-*N*Lys-*N*pm-*N*pm-*N*Lys-*N*pm-*N*pm-NH₂, H-*N*Lys-*N*pm- *N*pm-*N*Lys-*N*pm-*N*pm-*N*Lys-*N*spe-*N*pm-*N*Lys-*N*pm-*N*pm-NH₂, H-*N*Lys-*N*pm- *N*pm-*N*Lys-*N*pm-*N*pm-*N*Lys-*N*pm-*N*spe-*N*Lys-*N*pm-*N*pm-NH₂, H-*N*Lys-*N*pm- *N*pm-*N*Lys-*N*pm-*N*pm-*N*Lys-*N*pm-*N*pm-*N*Lys-spe-*N*pm-NH₂, H-*N*Lys-*N*pm- *N*pm-*N*Lys-*N*pm-*N*pm-*N*Lys-*N*pm-*N*pm-*N*Lys-*N*pm-*N*spe-NH₂, H-*N*Lys-*N*spe- *N*pm-*N*Lys-*N*pm-*N*pm-*N*Lys-*N*pm-*N*pm-*N*Lys-*N*pm-*N*spe-NH₂, H-*N*Lys-*N*spe- *N*spe-*N*Lys-*N*pm-*N*pm-*N*Lys-*N*pm-*N*pm-*N*Lys-*N*pm-*N*pm-NH₂, H-*N*Lys-Nspe- *N*spe-*N*Lys-*N*pm-*N*pm-*N*Lys-*N*pm-*N*pm-*N*Lys-*N*pm-*N*spe-NH₂, H-*N*Lys-*N*spe- *N*pm-*N*Lys-*N*pm-*N*pm-*N*Lys-*N*pm-*N*pm-*N*Lys-*N*spe-*N*spe-NH₂, H-*N*Lys-*N*pm- *N*pm-*N*Lys-*N*pm-*N*spe-*N*Lys-*N*spe-*N*spe-*N*Lys-*N*pm-*N*pm-NH₂, H-*N*Lys-*N*spe- *N*spe-*N*Lys-*N*pm-*N*pm-*N*Lys-*N*pm-*N*pm-*N*Lys-*N*spe-*N*spe-NH₂, and H-*N*Lys-*N*pm-*N*pm-*N*Lys-*N*spe-*N*spe-*N*Lys-*N*spe-*N*spe-*N*Lys-*N*pm-*N*pm-NH₂.

11. The biomedical device of claim 1, wherein said peptoid is a poly-N-substituted glycine compound of a formula 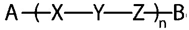 wherein
A is a terminal N-alkyl substituted glycine residue,
n is an integer,
B is selected from the group consisting of NH₂, one and two N-substituted glycine residues, and wherein said one and two N-substituted glycine residues have N-substituents which are independently selected from natural α-amino acid side chain moieties, isomers and carbon homologs thereof, and
X, Y and Z are independently selected from the group consisting of N-substituted glycine residues, wherein said N-substituents are independently selected from the group consisting of natural α-amino acid side chain moieties, isomers and carbon homologs thereof, and proline residues.

12. The biomedical device of claim 1, wherein said peptoid is a cyclic peptoid.

13. The biomedical device of claim 1, wherein the polyanionic microgel releases the peptoid in the presence of a pathogen selected from the group consisting of bacteria, fungi and viruses.

14. The biomedical device of claim 1, wherein the polyanionic microgel releases the peptoid in the presence of a pathogen selected from:
the group consisting of *Staphylococcus aureus, Pseudomonas aeruginosa, Escherichia coli, Klebsiella, Proteus, Enterobacter, Clostridium difficile,* and *Salmonela, Streptoccoci;* or
the group consisting of *Candida albicans, Aspergillus spp., Nocardia, Pneumocystis carinii, Cryptococcus neoformans,* and *Cryptosporidium;* or
the group consisting of respiratory syncytial virus, cytomegalovirus, human immunodeficiency virus (HIV), Ebola, rotavirus, enteroviruses, Influenza A (including subtypes H2N2 and H3N3), hepatitis and herpes viruses.

## Patentansprüche

1. Biomedizinische Vorrichtung, umfassend:
eine Oberfläche, die ausgewählt ist aus der Gruppe, bestehend aus Metalloberflächen, Keramikoberflächen und Polymeroberflächen;
ein polyanionisches Mikrogel, das auf der Oberfläche angeordnet ist; und
ein Peptoid, das in dem polyanionischen Mikrogel angeordnet ist;
wobei es sich bei der biomedizinischen Vorrichtung um eine Hüftprothese, eine Knieprothese, eine Herzklappe, einen Herzschrittmacher, ein Cochlea-Implantat, einen Shunt, ein chirurgisches Netz, eine Naht oder ein gewebebauliches Konstrukt handelt.

2. Biomedizinische Vorrichtung nach Anspruch 1, wobei die Oberfläche Folgendes umfasst:
mindestens ein Material, das aus der Gruppe ausgewählt ist, bestehend aus Metallen, Metalloxiden, Metalllegierungen und polymeren Materialien; oder
mindestens ein Material, das aus der Gruppe ausgewählt ist, bestehend aus Aluminiumoxid, Zirkoniumdioxid und zähhartem Aluminiumoxid (ZTA); oder
mindestens ein hochschmelzendes Metall; oder
mindestens eine Titanlegierung; oder
Edelstahl.

3. Biomedizinische Vorrichtung nach Anspruch 2, wobei die Oberfläche polymeres Material umfasst und das polymere Material Folgendes einschließt:
mindestens ein Material, das aus der Gruppe ausgewählt ist, bestehend aus Polyethylen, Polytetrafluorethylen (PTFE) und Polyetheretherketon (PEEK); oder
mindestens ein Material, das aus der Gruppe ausgewählt ist, bestehend aus ultrahochmolekularem Polyethylen (UHMWPE) und hochvernetztem Polyethylen (HXLPE).

4. Biomedizinische Vorrichtung nach Anspruch 2, wobei die Oberfläche mindestens eine Metalllegierung umfasst, die aus der Gruppe ausgewählt ist, bestehend aus Co-Cr-Mo-Legierungen und rostfreien Stahllegierungen.

5. Biomedizinische Vorrichtung nach Anspruch 2, wobei die Oberfläche ein hochschmelzendes Metall umfasst, das aus der Gruppe ausgewählt ist, bestehend aus Zr, Ta, V, Nb, W, Mo und deren Legierungen.

6. Biomedizinische Vorrichtung nach Anspruch 5, wobei die Oberfläche Folgendes umfasst:
eine Legierung, die aus der Gruppe ausgewählt ist, bestehend aus Ti-6Al-4V-Legierungen, Ti-5Al-2,5Fe-Legierungen, Ti-6Al-7Nb-Legierungen und β-Ti-Legierungen; oder
oxidiertes Zirkonium (Oxinium).

7. Biomedizinische Vorrichtung nach Anspruch 6, wobei die Oberfläche Ti-12Mo-6Zr-2Fe (TMZF) umfasst.

8. Biomedizinische Vorrichtung nach Anspruch 2, wobei die Oberfläche ein Material umfasst, das ausgewählt ist aus:
der Gruppe, bestehend aus Aluminiumoxid, Zirkoniumdioxid und zirkoniumdioxidverstärktem Aluminiumoxid; oder
der Gruppe, bestehend aus teilstabilisiertem Magnesiumoxid (MgPSZ) und stabilisierendem Yttriumoxid (Y-TZP).

9. Biomedizinische Vorrichtung nach Anspruch 1, wobei das Peptoid H-(NLys-Nspe-Nspe)₄-NH₂ ist.

10. Biomedizinische Vorrichtung nach Anspruch 1, wobei das Peptoid ausgewählt ist aus:
der Gruppe, bestehend aus: H-(*N*Lys-*N*spe-*N*spe)₄-NH₂, H-(*N*Lys-Nspe-Nspe(p-Br))₂-NH₂, H-*N*Lys-*N*spe-*N*spe-*N*Lys-*N*spe-*N*spe(p-Br)-NH₂, H-((*N*Lys-*N*spe(p-Br)-*N*spe(p-Br))₂-NH₂, H-*N*tridec-*N*Lys-*N*spe-*N*spe-*N*Lys-NH₂, H-(*N*Lys-*N*spe-*N*spe)₃-*N*Lys-*N*spe*-N*H₂, H-(*N*Lys-*N*spe-*N*spe)₂-NH₂, H-*N*dec- (*N*Lys-*N*spe-*N*spe)₂-NH₂, H-*N*dec-(*N*Lys-*N*spe-*N*spe(p-Br))₂-NH₂, H-*N*tridec-(*N*Lys-*N*spe-*N*spe(p-Br))₂-NH₂, H- (NLys-*N*spe-*N*spe)₄-*N*Lys-NH₂, H-(*N*Lys-*N*spe-*N*spe(p-Br))₂-*N*Lys-NH₂, H-*N*Lys-*N*spe-*N*spe-*N*Lys-*N*spe-*N*spe(p-Br)-*N*Lys-NH₂, H- (*N*Lys-*N*spe(p-Br)-*N*spe(p-Br))₂-*N*Lys-NH₂, H-*N*tridec-*N*Lys-*N*spe-*N*spe-*N*Lys-*N*Lys-NH₂, H- (*N*Lys-*N*spe-*N*spe)₃-*N*lys-*N*spe-*N*Lys-NH₂, H- (*N*Lys-*N*spe-*N*spe)₂-*N*Lys-NH₂, H-*N*dec- (*N*Lys-*N*spe-*N*spe)₂-*N*Lys-NH₂, H-*N*dec-(*N*Lys-*N*spe-*N*spe(p-Br))₂-*N*Lys-NH₂, H-*N*tridec-(*N*Lys-*N*spe-*N*spe(p-Br))₂-*N*Lys-NH₂, und H- (*N*Lys-*N*ssb-*N*ssb)₄-NH₂; oder
der Gruppe, bestehend aus: H-(*N*Lys-*N*spe-*N*spe(p-Br))₂-NH₂, H-*N*Lys-*N*spe-*N*spe-*N*Lys-*N*spe-*N*spe(p-Br)-NH₂, H-((*N*lys-*N*spe(p-Br)*N*spe(p-Br))₂-NH₂, H-*N*dec-(*N*Lys-*N*spe-*N*spe (p-Br))₂-NH₂, H-*N*tridec-(*N*Lys-*N*spe-*N*spe(p-Br))₂-NH₂, H-(*N*Lys-*N*spe-*N*spe(p-Br))₂-*N*Lys-NH₂, H-*N*Lys-*N*spe-*N*spe-*N*Lys-*N*spe-*N*spe(p-Br)-*N*Lys-NH₂, H-(*N*Lys-Nspe(p-Br)-*N*spe(p-Br))₂-*N*Lys-NH₂, H-*N*dec-(*N*Lys-*N*spe-*N*spe(p-Br))₂-*N*Lys-NH₂, und H-*N*tridec-(*N*Lys-*N*spe-*N*spe(p-Br))₂-*N*Lys-NH₂; oder
der Gruppe, bestehend aus: H-*N*Lys-*N*spe-*N*spe-*N*Lys-*N*spe-*N*spe-*N*Lys-*N*spe-*N*spe-*N*Lys-*N*spe-*N*spe-NH₂, H-*N*Lys-*N*prn-*N*prn-*N*Lys-*N*pm-*N*pm-*N*Lys-*N*pm-*N*pm-*N*Lys-*N*pm-*N*pm-NH₂, H-*N*Lys-*N*spe-*N*pm-*N*Lys-*N*pm-*N*pm-*N*Lys-*N*pm-*N*pm-*N*Lys-*N*pm-*N*pm-NH₂, H-*N*Lys-*N*pm-*N*spe-*N*Lys-*N*pm-Npm-*N*Lys-*N*pm-*N*pm-*N*Lys-*N*pm-*N*pm-NH₂, H-*N*Lys-*N*prn-*N*prn-*N*Lys-*N*spe-*N*prn-*N*Lys-*N*prn-*N*prn-*N*Lys-*N*prn-*N*prn-NH₂, H-*N*Lys-*N*prn-*N*prn-*N*Lys-*N*prn-*N*spe-*N*Lys-*N*prn-*N*prn-*N*Lys-*N*prn-*N*pm-NH₂, H-*N*Lys-*N*prn-*N*prn-*N*Lys-*N*prn-*N*prn-*N*Lys-*N*spe-*N*prn-*N*Lys-*N*pm-*N*pm-NH₂, H-*N*Lys-*N*prn-*N*prn-*N*Lys-*N*prn-*N*prn-*N*Lys-*N*prn-*N*spe-*N*Lys-*N*pm-*N*pm-NH₂, H-*N*Lys-*N*prn-*N*prn-*N*Lys-*N*prn-*N*prn-*N*Lys-*N*prn-*N*pm-*N*Lys-spe-*N*pm-NH₂, H-*N*Lys-*N*prn-*N*prn-*N*Lys-*N*prn-*N*prn-*N*Lys-*N*pm-*N*pm-*N*Lys-*N*pm-*N*spe-NH₂, H-*N*Lys-*N*spe-*N*prn-*N*Lys-*N*prn-*N*prn-*N*Lys-*N*pm-*N*pm-*N*Lys-*N*pm-*N*spe-NH₂, H-*N*Lys-*N*spe-*N*spe-*N*Lys-*N*pm-*N*pm-*N*Lys-*N*pm-*N*pm-*N*Lys-*N*pm-*N*pm-NH₂, H-*N*Lys-*N*spe-*N*spe-*N*Lys-*N*pm-*N*pm-*N*Lys-*N*pm-*N*pm-*N*Lys-*N*pm-*N*spe-NH₂, H-*N*Lys-*N*spe-*N*pm-*N*Lys-*N*pm-*N*pm-*N*Lys-*N*pm-*N*pm-*N*Lys-*N*spe-*N*spe-NH₂, H-*N*Lys-*N*prn-*N*prn-*N*Lys-*N*prn-*N*spe-*N*Lys-*N*spe-*N*spe-*N*Lys-*N*prn-*N*pm-NH₂, H-*N*Lys-*N*spe-*N*spe-*N*Lys-*N*pm-*N*pm-*N*Lys-*N*pm-*N*pm-*N*Lys-*N*spe-*N*spe-NH₂, und *H*-NLys-*N*pm-*N*pm-*N*Lys-*N*spe-*N*spe-*N*Lys-*N*spe-*N*spe-*N*Lys-*N*pm-*N*pm-NH₂.

11. Biomedizinische Vorrichtung nach Anspruch 1, wobei das Peptoid eine Poly-N-substituierte Glycinverbindung mit der folgenden Formel ist 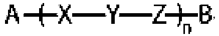 wobei
A ein terminaler N-alkylsubstituierter Glycinrest ist,
n eine ganze Zahl ist,
B ausgewählt ist aus der Gruppe, bestehend aus NH₂, einem und zwei N-substituierten Glycinresten, und wobei die ein und zwei N-substituierten Glycinreste N-Substituenten aufweisen, die unabhängig voneinander ausgewählt sind aus natürlichen α-Aminosäure-Seitenkettenanteilen, Isomeren und Kohlenstoffhomologen davon, und
X, Y und Z unabhängig voneinander aus der Gruppe ausgewählt sind, bestehend aus N-substituierten Glycinresten, wobei die N-Substituenten unabhängig voneinander aus der Gruppe ausgewählt sind, bestehend aus natürlichen α-Aminosäure-Seitenkettenanteilen, Isomeren und Kohlenstoffhomologen davon und Prolinresten.

12. Biomedizinische Vorrichtung nach Anspruch 1, wobei das Peptoid ein zyklisches Peptoid ist.

13. Biomedizinische Vorrichtung nach Anspruch 1, wobei das polyanionische Mikrogel das Peptoid in Gegenwart eines Erregers freisetzt, der aus der Gruppe ausgewählt ist, bestehend aus Bakterien, Pilzen und Viren.

14. Biomedizinische Vorrichtung nach Anspruch 1, wobei das polyanionische Mikrogel das Peptoid in Gegenwart eines Pathogens freisetzt, das ausgewählt ist aus:
der Gruppe, bestehend aus *Staphylococcus aureus, Pseudomonas aeruginosa, Escherichia coli, Klebsiella, Proteus, Enterobacter, Clostridium difficile* und *Salmonela, Streptoccoci;* oder
der Gruppe, bestehend aus *Candida albicans, Aspergillus spp., Nocardia, Pneumocystis carinii, Cryptococcus neoformans* und *Cryptosporidium;* oder
der Gruppe, bestehend aus respiratorischem Synzytialvirus, Cytomegalovirus, humanem Immundefizienzvirus (HIV), Ebola, Rotavirus, Enteroviren, Influenza A (einschließlich der Subtypen H2N2 und H3N3), Hepatitis- und Herpesviren.

## Revendications

1. Dispositif biomédical, comprenant :
une surface sélectionnée parmi le groupe constitué de surfaces métalliques, de surfaces céramiques et de surfaces polimériques ;
un microgel polyanionique disposé sur ladite surface ; et
un peptoïde disposé dans ledit microgel polyanionique ;
dans lequel le dispositif biomédical est une prothèse de hanche, une prothèse de genou, une valve cardiaque, un stimulateur cardiaque, un implant cochléaire, une dérivation, un treillis chirurgical, une suture ou une construction d'ingénierie tissulaire.

2. Dispositif biomédical selon la revendication 1, dans lequel ladite surface comprend :
au moins un matériau sélectionné parmi le groupe constitué de métaux, d'oxydes métalliques, d'alliages métalliques et de matériaux polymériques ; ou
au moins un matériau sélectionné parmi le groupe constitué d'alumine, de zircone et d'alumine renforcée à la zircone (ZTA) ; ou
au moins un métal réfractaire ; ou
au moins un alliage de titane ; ou
de l'acier inoxydable.

3. Dispositif biomédical selon la revendication 2, dans lequel ladite surface comprend un matériau polymérique, et ledit matériau polymérique comporte :
au moins un matériau sélectionné parmi le groupe constitué de polyéthylène, de polytétrafluoroéthylène (PTFE) et de polyétheréthercétone (PEEK) ; ou
au moins un matériau sélectionné parmi le groupe constitué de polyéthylène de poids moléculaire ultra-élevé (UHMWPE) et de polyéthylène hautement réticulé (HXLPE).

4. Dispositif biomédical selon la revendication 2, dans lequel ladite surface comprend au moins un alliage métallique sélectionné parmi le groupe constitué d'alliages Co-Cr-Mo et d'alliages d'acier inoxydable.

5. Dispositif biomédical selon la revendication 2, dans lequel ladite surface comprend un métal réfractaire sélectionné parmi le groupe constitué de Zr, Ta, V, Nb, W, Mo et d'alliages de ceux-ci.

6. Dispositif biomédical selon la revendication 5, dans lequel ladite surface comprend :
un alliage sélectionné parmi le groupe constitué d'alliages Ti-6Al-4V, d'alliages Ti-5Al-2,5Fe, d'alliages Ti-6Al-7Nb et d'alliages β-Ti ; ou
du zirconium oxydé (oxinium).

7. Dispositif biomédical selon la revendication 6, dans lequel ladite surface comprend du Ti-12Mo-6Zr-2Fe (TMZF).

8. Dispositif biomédical selon la revendication 2, dans lequel ladite surface comprend un matériau parmi :
le groupe constitué d'alumine, de zircone et d'alumine renforcée à la zircone ; ou
le groupe constitué de zircone partiellement stabilisée à la magnésie (MgPSZ) et d'oxyde stabilisé à l'yttrium (Y-TZP).

9. Dispositif biomédical selon la revendication 1, dans lequel ledit peptoïde est H-(NLys-Nspe-Nspe)₄-NH₂.

10. Dispositif biomédical selon la revendication 1, dans lequel ledit peptoïde est sélectionné parmi :
le groupe constitué de : H-(*N*Lys-*N*spe-*N*spe)₄-NH₂, H-(*N*Lys-*N*spe-*N*spe(p-Br))₂-NH₂, H-*N*Lys-*N*spe-*N*spe-*N*Lys-*N*spe-*N*spe(p-Br)-NH₂, H-((*N*Lys-*N*spe(p-Br)-*N*spe(p-Br))₂-NH₂, H-*N*tridec-NLys-Nspe-Nspe-NLys-NH₂, H-(*N*Lys-*N*spe-*N*spe)₃-*N*Lys-*N*spe-NH₂, H- (*N*Lys-*N*spe-*N*spe)₂-NH₂, H-*N*dec-(*N*Lys-*N*spe-*N*spe)₂-NH₂, H-*N*dec-(*N*Lys-*N*spe-*N*spe(p-Br))₂-NH₂, H-*N*tridec-(*N*Lys-*N*spe-*N*spe(p-Br))₂-NH₂, H-(*N*Lys-*N*spe-*N*spe)₄-*N*Lys-NH₂, H-(*N*Lys-*N*spe-*N*spe(p-Br))₂-*N*Lys-NH₂, H-*N*Lys-*N*spe-*N*spe-*N*Lys-*N*spe-*N*spe(p-Br)-*N*Lys-NH₂, H-(*N*Lys-*N*spe(p-Br)-*N*spe(p-Br))₂-*N*Lys-NH₂, H-*N*tridec-*N*Lys-*N*spe-*N*spe-*N*Lys-*N*Lys-NH₂, H-(*N*Lys-*N*spe-*N*spe)₃-*N*lys-*N*spe-*N*Lys-NH₂, H-(*N*Lys-*N*spe-*N*spe)₂-*N*Lys-NH₂, *H*-Ndec-(*N*Lys-*N*spe-*N*spe)₂-*N*Lys-NH₂, H-*N*dec-(*N*Lys-*N*spe-*N*spe(p-Br)) ₂-*N*Lys-NH₂, H-*N*tridec-(*N*Lys-*N*spe-*N*spe(p-Br))₂-*N*Lys-NH₂, et H-(*N*Lys-*N*ssb-*N*ssb)₄-NH₂ ; ou
le groupe constitué de : H-(*N*Lys-*N*spe-*N*spe(p-Br))₂-NH₂, H-*N*Lys-*N*spe-*N*spe-*N*Lys-*N*spe-*N*spe(p-Br)-NH₂, H-((*N*Lys-*N*spe(p-Br)-*N*spe(p-Br))₂-NH₂, H-*N*dec-(*N*Lys-*N*spe-*N*spe (p-Br))₂-NH₂, H-*N*tridec-(*N*Lys-*N*spe-*N*spe(p-Br))₂-NH₂, H-(*N*Lys-*N*spe-*N*spe(p-Br))₂-*N*Lys-NH₂, H-*N*Lys-*N*spe-*N*spe-*N*Lys-*N*spe-*N*spe(p-Br)-*N*Lys-*N*H₂, H-(*N*Lys-*N*spe(p-Br)-*N*spe(p-Br))₂-*N*Lys-NH₂, H-*N*dec-(*N*Lys-*N*spe-*N*spe(p-Br))₂-*N*Lys-NH₂, et H-*N*tridec-(*N*Lys-*N*spe-*N*spe(p-Br))₂-*N*Lys-*N*H₂ ; ou
le groupe constitué de : H-*N*Lys-*N*spe-*N*spe-*N*Lys-*N*spe-*N*spe-*N*Lys-*N*spe-*N*spe-*N*Lys-*N*spe-*N*spe-NH₂, H-*N*Lys-*N*pm-*N*pm-*N*Lys-*N*pm-*N*pm-*N*Lys-*N*pm-*N*pm-*N*Lys-*N*pm-*N*pm-NH₂, H-*N*Lys-*N*spe-*N*pm-*N*Lys-*N*pm-*N*pm-*N*Lys-*N*pm-*N*pm-*N*Lys-*N*pm-*N*pm-NH₂, H-*N*Lys-*N*pm-*N*spe-*N*Lys-*N*pm-*N*pm-*N*Lys-*N*pm-*N*pm-*N*Lys-*N*pm-*N*pm-NH₂, H-*N*Lys-*N*pm-*N*pm-*N*Lys-*N*spe-*N*pm-*N*Lys-*N*pm-*N*pm-*N*Lys-*N*pm-*N*pm-NH₂, H-*N*Lys-*N*pm-*N*pm-*N*Lys-*N*pm-*N*spe-*N*Lys-*N*pm-*N*pm-*N*Lys-*N*pm-*N*pm-NH₂, H-*N*Lys-*N*pm-*N*pm-*N*Lys-*N*pm-*N*pm-*N*Lys-*N*spe-*N*pm-*N*Lys-*N*pm-*N*pm-NH₂, H-*N*Lys-*N*pm-*N*pm-*N*Lys-*N*pm-*N*pm-*N*Lys-*N*pm-*N*spe-*N*Lys-*N*pm-*N*pm-NH₂, H-*N*Lys-*N*pm-*N*pm-*N*Lys-*N*pm-*N*pm-*N*Lys-*N*pm-*N*pm-*N*Lys-spe-*N*pm-NH₂, H-*N*Lys-*N*pm-*N*pm-*N*Lys-*N*pm-*N*pm-*N*Lys-*N*pm-*N*pm-*N*Lys-*N*pm-*N*spe-NH₂, H-*N*Lys-*N*spe-*N*pm-*N*Lys-*N*pm-*N*pm-*N*Lys-*N*pm-*N*pm-*N*Lys-*N*pm-*N*spe-NH₂: H-*N*Lys-*N*spe-*N*spe-*N*Lys-*N*pm-*N*pm-*N*Lys-*N*pm-*N*pm-*N*Lys-*N*pm-*N*pm-NH₂, H-*N*Lys-*N*spe-*N*spe-*N*Lys-*N*pm-*N*pm-*N*Lys-*N*pm-*N*pm-*N*Lys-*N*pm-*N*spe-NH₂, H-*N*Lys-*N*spe-*N*pm-*N*Lys-*N*pm-*N*pm-*N*Lys-*N*pm-*N*pm-*N*Lys-*N*spe-*N*spe-NH₂, H-*N*Lys-*N*pm-*N*pm-*N*Lys-*N*pm-*N*spe-*N*Lys-*N*spe-*N*spe-*N*Lys-*N*pm-*N*pm-NH₂, H-*N*Lys-*N*spe-*N*spe-*N*Lys-*N*pm-*N*pm-*N*Lys-*N*pm-*N*pm-*N*Lys-*N*spe-*N*spe-NH₂, et H*-N*Lys-*N*pm-*N*pm-*N*Lys-*N*spe-*N*spe-*N*Lys-*N*spe-*N*spe-*N*Lys-*N*pm-*N*pm-NH₂.

11. Dispositif biomédical selon la revendication 1, dans lequel ledit peptoïde est un composé de glycine poly-N-substituée d'une formule 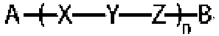 où
A est un résidu de glycine N-alkyl substitué en position terminale,
n est un nombre entier,
B est sélectionné parmi le groupe constitué de NH₂, un et deux résidus de glycine N-substitués, et dans lequel lesdits un et deux résidus de glycine N-substitués ont des substituants N qui sont sélectionnés indépendamment parmi les fragments de chaînes latérales d'a-acides aminés naturels, les isomères et homologues carbonés de ceux-ci, et
X, Y et Z sont sélectionnés indépendamment parmi le groupe constitué de résidus de glycine N-substitués, dans lequel lesdits substituants N sont sélectionnés indépendamment parmi le groupe constitué de fragments de chaînes latérales d'α-acides aminés naturels, les isomères et homologues carbonés de ceux-ci, et les résidus de proline.

12. Dispositif biomédical selon la revendication 1, dans lequel ledit peptoïde est un peptoïde cyclique.

13. Dispositif biomédical selon la revendication 1, dans lequel le microgel polyanionique libère le peptoïde en présence d'un agent pathogène sélectionné parmi le groupe constitué de bactéries, de champignons et de virus.

14. Dispositif biomédical selon la revendication 1, dans lequel le microgel polyanionique libère le peptoïde en présence d'un agent pathogène sélectionné parmi :
le groupe constitué de *Staphylococcus aureus,* de *Pseudomonas aeruginosa,* d'*Escherichia coli,* de *Klebsiella,* de *Proteus,* d'*Enterobacter,* de *Clostridium difficile,* et de *Salmonela,* de *Streptoccoci* ; ou
le groupe constitué de *Candida albicans,* de *Aspergillus spp.*, de *Nocardia,* de *Pneumocystis carinii,* de *Cryptococcus neoformans,* et de *Cryptosporidium ;* ou
le groupe constitué de virus respiratoire syncytial, de cytomégalovirus, de virus de l'immunodéficience humaine (VIH), d'Ebola, de rotavirus, d'entérovirus, de grippe A (y compris les sous-types H2N2 et H3N3), de virus de l'hépatite et de l'herpès.
